(19) Europäisches Patentamt
European Patent Office
Office européen des brevets

(11) Publication number: **0 234 494 B1**

(12) # EUROPEAN PATENT SPECIFICATION

(45) Date of publication of patent specification: **13.03.91**

(51) Int. Cl.⁵: **C07D 493/04**, A61K 31/505, A61K 31/53, A61K 31/41, //(C07D493/04,307:00,307:00)

(21) Application number: **87102295.0**

(22) Date of filing: **18.02.87**

The file contains technical information submitted after the application was filed and not included in this specification

(54) **Isohexide nucleosides, processes for their preparation and their use as medicaments.**

(30) Priority: **28.02.86 DE 3606634**

(43) Date of publication of application: **02.09.87 Bulletin 87/36**

(45) Publication of the grant of the patent: **13.03.91 Bulletin 91/11**

(84) Designated Contracting States: **AT BE CH DE ES FR GB GR IT LI LU NL SE**

(56) References cited:
**EP-A- 0 167 008**
**AU-B- 547 387**
**FR-M- 4 141**
**GB-A- 1 027 891**
**US-A- 4 169 152**

(73) Proprietor: **Heinrich Mack Nachf.**
**Postfach 2064**
**W-7918 Illertissen(DE)**

(72) Inventor: **Stoss, Peter, Dr.Dipl.-Chem.**
**Mozartstrasse 15**
**W-7918 Illertissen(DE)**
Inventor: **Kaes, Elmar**
**Birkenweg 16**
**W-7918 Illertissen(DE)**

(74) Representative: **Lederer, Franz, Dr. et al**
**Lederer, Keller & Riederer Patentanwälte**
**Lucile-Grahn-Strasse 22**
**W-8000 München 80(DE)**

**EP 0 234 494 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel isohexide nucleosides, processes for their preparation and their use as medicaments, in particular as virustatics

The isohexide class of compound has been known for about 100 years. A. Fauconnier, Bull. Soc. Chim. France 41, 119 (1884) described isomannide as the first representative. This is attributed a diuretic action, as is also isosorbide, which became known later; see, for example, Proc. Soc. exp. Biol. Med. 119, 39 1965) and U.S. Patent 2,143,324. A number of derivatives have since been recognized as pharmacologically active. Of these, isosorbide 2,5-dinitrate has been on the market for many years and isosorbide 5-mononitrate since a short time as coronary therapeutics.

German Offenlegungsschrift 2,221,080 relating to the mononitrate esters of isosorbide, which can be used for the treatment of angina pectoris, also belongs to the prior art. British Patent Specification 1,027,891, describes nicotinic acid esters of isohexides with vasodilatory properties. Isohexide ethers with a muscle-relaxing action are mentioned, inter alia, in U.S. Patent 4,169,152. 0-Substituted isohexide mononitrates with an action on the cardiovascular system are the subject matter of German Offenlegungsschrift 3,028,289. German Offenlegungsschrift 3,248,584 describes acyl derivatives of isohexides with a cardiovascular action.

German Offenlegungsschrift 3,421,072 relates to isohexide derivatives for which both cardiovascular activities and spasmolytic and antitussive activities are claimed. FR-M-4141 discloses 2,5-di-0-acetyl-d-glucosaccharo-1,4,3-6-dilactone as medicament with ß-glucuronidase inhibiting activity.

As well as isohexide derivatives, various deoxy compounds with a pharmacological action, in which the oxygen in the 2- or 5-position is replaced by an optionally substituted amino group, are known. Although these are no longer isohexides in the actual sense, they are also mentioned here for completeness: J. Indian Chem. 16 B, 153 (1978), German Offenlegungsschrift 3,028,272, German Offenlegungsschrift 3,028,273, German Offenlegungsschrift 3,028,288 and German Offenlegungsschrift 3,028,340.

Finally, compounds which no longer contain free or substituted hydroxyl groups have also been prepared, in particular in accordance with Carbohydr. Res. 85, 259 (1980) and German Offenlegungsschrift 3,109,532, where 2,5-diazido-2,5-dideoxy derivatives with a hypnotic action are mentioned.

The isohexide derivatives according to the invention differ structurally from all these compounds which have already been described in that for the first time they contain a nucleosidic bond with oxygen-containing heterocyclic radicals as a characteristic structural feature. Such compounds are novel. It was not to be predicted that the substances of the present invention are moreover distinguished by novel and useful pharmacological properties. Needless to say, none of the isohexide derivatives already described has an action spectrum which is comparable to that of the compounds disclosed here.

The present invention therefore relates to novel isohexide nucleosides of the general formula I

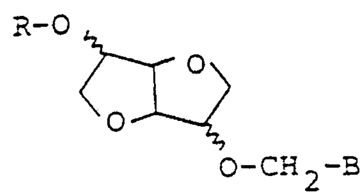

I

in which the bond between the ring system and the substituents can be either endo- or exo-cyclic in position, and in which R denotes hydrogen, a straight-chain or branched aliphatic acyl radical with 2 to 5 carbon atoms, preferably such a radical with 2 to 3 carbon atoms, in particular an acetyl radical, an aromatic acyl radical, preferably an unsubstituted or substituted benzoyl radical, it being possible for the substituents to be halogens, lower alkyl groups or a nitro group, in particular a benzoyl, toluyl, chlorobenzoyl or nitrobenzoyl radical, a benzyl radical or a phosphate radical and B represents an organic base radical from the group comprising nitrogen-containing heterocyclic radicals, in particular those with more than one hetero atom, preferably from the group comprising 5- and 6-membered nitrogen-heterocyclic radicals, in particular from the series comprising pyrimidine, triazine, triazole and imidazole derivatives, specifically

a) uracils of the general formula II

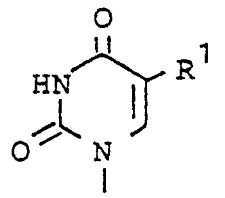

II

wherein $R^1$ denotes hydrogen, a halogen radical or a straight-chain or branched alkyl, alkenyl or alkynyl radical which has 1 to 6 carbon atoms and is optionally substituted by hydroxyl groups or one or more halogens, the expression halogen in each case representing fluorine, chlorine, bromine or iodine,

b) cytosines of the general formula III

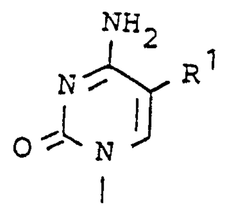

III

in which $R^1$ has the abovementioned meaning,

c) isocytosines of the general formula IV

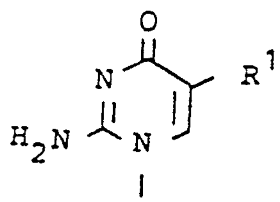

IV

in which $R^1$ has the abovementioned meaning,

d) 5-azacytosine of the formula V

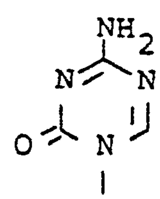

V

e) triazoles of the general formula VI

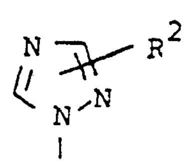

VI

in which the substituent $R^2$ occupies either the 3- or the 5-position and has the following meaning: hydrogen, an alkoxycarbonyl radical $COOR^3$, in which $R^3$ denotes hydrogen, a straight-chain or branched alkyl group with 1 to 5 carbon atoms, a carboxamide radical $CO-NH_2$, a thiocarboxamide radical $CS-NH_2$ or a cyano radical CN,

f) imidazoles of the general formula VII

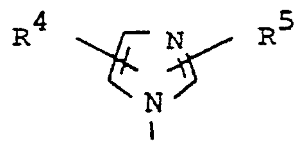

VII

in which the two substituents $R^4$ and $R^5$ are identical or different and denote hydrogen, an amino radical $NH_2$, a carboxamide radical $CO\text{-}NH_2$, a thiocarboxamide radical $CS\text{-}NH_2$ or a cyano radical CN, and, if appropriate, salts of compounds of the general formula I with inorganic and organic acids, preferably with pharmaceutically acceptable acids.

Unless stated otherwise, lower alkyl groups designate those with up to 6 carbon atoms.

According to a preferred embodiment, the isohexide nucleosides of the general formula I contain, as the radical R, hydrogen or a phosphate radical and, as the radical B, the uracils of the general formula II, the cytosines of the general formula III, the isocytosines of the general formula IV, the radical $R^1$ in each case representing hydrogen, methyl, ethyl, propyl, isopropyl, butyl, isobutyl, pentyl, hexyl, hydroxymethyl, hydroxyethyl, vinyl, allyl, ethynyl, fluorine, chlorine, bromine, iodine, bromovinyl, iodovinyl or trifluoromethyl, or 5-azacytosine of the formula V, the triazoles of the general formula VI, in which $R^2$ represents the carboxamide radical, the methoxycarbonyl radical or the ethoxycarbonyl radical, or the imidazoles of the general formula VII, in which one of the substituents $R^4$ and $R^5$ denotes an amino radical $NH_2$ ahd the other denotes a carboxamide radical $CO\text{-}NH_2$ or s cyano radical CN, and, if appropriate, salts thereof with inorganic and organic acids, preferably with pharmaceutically acceptable acids.

Isohexides consist of two cis-linked, almost flat tetrahydrofuran rings which form an angle of about 120° with one another. Each of the tetrahydrofuran rings carries a hydroxyl group. This can occupy an endo- or an exo-position, depending on the orientation to the ring system. Four isomeric forms of the compounds of the general formula I according to the invention consequently exist, and in particular isomannide nucleosides of the general formula Ia

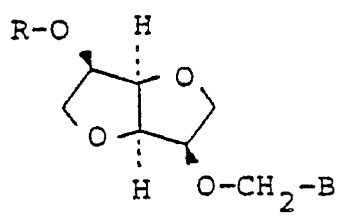

Ia

in which the two ring substituents in the 2- and 5-position occupy the endo-position, R and B having the abovementioned meaning, isoidide nucleosides of the general formula Ib

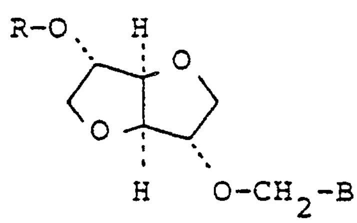

Ib

in which the two ring substituents in the 2- and 5-position occupy the exo-position, R and B having the abovementioned meaning, and isosorbide nucleosides of the general formulae Ic and Id

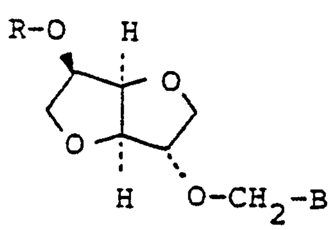

Ic

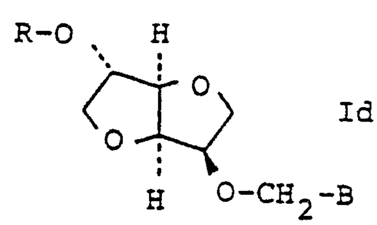

Id

in which one of the two ring substituents in the 2- and 5-position in each case occupies the endo-position and the other occupies the exo-position, R and B having the abovementioned meaning.

By convention, the exo-position of isosorbide derivatives is designated position 2 and the endo-position is designated position 5. In contrast, differentiation between positions 2 and 5 is not possible in the case of isomannide and isoidide derivatives. A brief summary of the stereochemistry of the isohexides is given by J.A. Mills in Advances in Carbohydrate Chemistry 10, 1-53 (1965) and L. Hough and A.C. Richardson in Rodd's Chemistry of Carbon Compounds, 2nd edition Volume 1, F, Elsevier, 1967, pages 51-55.

As well as the designation isohexides selected here, the names 1,4:3,6-dianhydro-hexites, 1,4:3,6-dianhydro-hexitols and isohexitols are also used for the fundamental ring system. According to systematic nomenclature, the compounds are shown as bridge ring systems with the designation 2,6-dioxabicyclo-[3.3.0]octane-4,8-diols, and as fused systems with the designation hexahydrofuro[3,2-b]furan-3,6-diols. One of the above names can therefore also be used for the compounds according to the invention.

The invention also relates to processes for the preparation of compounds of the general formula I. The isohexide nucleosides presented here can in principle be synthesized by any process suitable for these compounds. One of these possibilities is shown by way of example in the following equation:

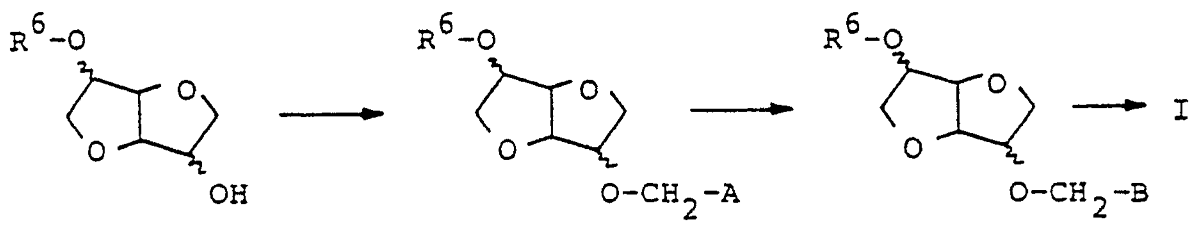

VIII IX X

According to this equation, the compounds according to the invention are obtainable in a manner which is known per se by converting a monosubstituted isohexide of the general formula VIII in which $R^6$ represents one of the protective groups customary in nucleoside chemistry, and in particular represents a straight-chain or branched aliphatic acyl radical with 2 to 5 carbon atoms, preferably such an acyl radical with 2 or 3 carbon atoms, in particular an acetyl radical, or represents an aromatic acyl radical, preferably an unsubstituted or substituted benzoyl radical, it being possible for the substituents to be halogens, lower alkyl groups or a nitro group, in particular a benzoyl, toluoyl, chlorobenzoyl or nitrobenzoyl radical, or represents a benzyl radical, into a compound of the general formula IX in which $R^6$ has the abovementioned meaning and A represents a leaving group or a leaving atom, and represents a halogen radical, in particular a chlorine, bromine or iodine radical, or represents an acyloxy radical, the expression "acyl" comprising lower straight-chain or branched aliphatic acyl groups with 2 to 5 carbon atoms, but preferably acetyl.

Such reactions can be carried out in a manner which is known per se. In the case where A denotes a halogen radical, compounds of the general formula VIII are subjected to halogenomethylation, which is usually carried out by means of formaldehyde and hydrogen halide. The formaldehyde can be used here either as an aqueous solution or in solid polymeric form. The hydrogen halide can also be used either in anhydrous form or in the form of aqueous solutions. Depending on the method chosen, a suitable solvent is used, such as water, methylene chloride, dichloroethane, trichloroethane, chloroform, diethyl ether, diisopropyl ether, tetrahydrofuran, dioxane, benzene, toluene or xylene, or mixtures of the above solvents. The reaction temperatures are in the range between -50° and +50°C, preferably in the range between -10°C and room temperature, over reaction times of 0.1 to 24 hours. Certain halogenomethyl compounds can of course also be converted into other halogenomethyl compounds. Thus, for example, the chloromethyl compounds can easily be converted into bromomethyl and iodomethyl compounds by means of known methods.

Compounds of the general formula IX in which A represents an acyloxy radical can be obtained in a manner which is known per se from the corresponding halogenomethyl derivatives by reaction with alkali metal acylates. Possible alkali metal acylates are chiefly sodium and potassium acylates, preferably sodium acetate and potassium acetate. Solvents are usually employed here, such as dimethylformamide, dimethylacetamide, dimethyl sulfoxide, hexamethylphosphoric acid triamide, sulfolane, tetrahydrofuran, dioxane, acetonitrile and acetone, at temperatures between -50 and +50°C, preferably between -10°C and room temperature, over reaction times between 1 and 24 hours.

The compounds of the general formula VIII employed as starting substances are known in mose cases

or can be prepared by known methods. On the other hand, the compounds of the general formula IX are novel intermediates. The following may be mentioned as examples of some of these novel intermediates: 5-0-acetyl-2-0-chloromethyl-isosorbide, 2-0-acetyl-5-0-chloromethyl-isosorbide, 5-0-acetyl-2-0-chloromethyl-isomannide, 5-0-acetyl-2-0-chloromethyl-isoidide, 5-0-benzoyl-2-0-chloromethyl-isosorbide, 2-0-benzoyl-5-0-chloromethyl-isosorbide, 5-0-benzoyl-2-0-chloromethyl-isomannide, 5-0-benzoyl-2-0-chloromethyl-isoidide, 5-0-(4-chlorobenzoyl)-2-0-chloromethyl-isosorbide, 2-0-(4-chlorobenzoyl)-5-0-chloromethyl-isosorbide, 5-0-(4-chlorobenzoyl)-2-0-chloromethyl-isomannide, 5-0-(4-chlorobenzoyl)-2-0- chloromethyl-isoidide, 2-0-chloromethyl-5-0-(4-toluoyl)-isosorbide, 5-0-chloromethyl-2-O-(4-toluoyl)-isosorbide, 2-0-chloromethyl-5-0-(4-toluoyl)-isomannide, 2-0-chloromethyl-5-0-(4-toluoyl)-isoidide, 2-0-chloromethyl-5-0-(2-nitrobenzoyl)-isosorbide, 5-0-chloromethyl-2-0-(2-nitrobenzoyl)-isosorbide, 2-0-chloromethyl-5-0-(2-nitrobenzoyl)-isomannide, 2-0-chloromethyl-5-0-(2-nitrobenzoyl)-isoidide, 2-0-benzyl-5-0-chloromethyl-isosorbide, 5-0-benzyl-2-0-chloromethyl-isosorbide, 5-0-benzyl-2-0-chloromethyl-isomannide, 5-0-benzyl-2-0-chloromethylisoidide, 2-0-acetoxymethyl-5-0-acetyl-isosorbide, 5-0-acetoxymethyl-2-0-acetyl-isosorbide, 2-0-acetoxymethyl-5-0-acetyl-isomannide, 2-0-acetoxymethyl-5-0-acetyl-isoidide, 2-0-acetoxymethyl-5-0-benzoyl-isosorbide, 5-0-acetoxymethyl-2-0-benzoyl-isosorbide, 2-0-acetoxymethyl-5-0-benzoyl-isomannide, 2-0-acetoxymethyl-5-0-benzoyl-isoidide, 2-0-acetoxymethyl-5-0-(4-chlorobenzoyl)-isosorbide, 5-0-acetoxymethyl-2-0-(4-chlorobenzoyl)-isosorbide, 2-0-acetoxymethyl-5-0-(4-chlorobenzoyl)-isomannide, 2-0-acetoxymethyl-5-0-(4-chlorobenzoyl)-isoidide, 2-0-acetoxymethyl-5-0-(4-toluoyl)-isosorbide, 5-0-acetoxymethyl-2-0-(4-toluoyl)-isosorbide, 2-0-acetoxymethyl-5-0-(4-toluyl)-isomannide, 2-0-acetoxymethyl-5-0-(4-toluoyl)-isoidide, 2-0-acetoxymethyl-5-0-(2-nitrobenzoyl)-isosorbide, 5-0-acetoxymethyl-2-0-(2-nitrobenzoyl)-isosorbide, 2-0-acetoxymethyl-5-0-(2-nitrobenzoyl)-isomannide, 2-0-acetoxymethyl-5-0-(2-nitrobenzoyl)-isoidide, 2-0-acetoxymethyl-5-0-benzyl-isosorbide, 5-0-acetoxymethyl-2-0-benzyl-isosorbide, 2-0-acetoxymethyl-5-0-benzyl-isomannide and 2-0-acetoxymethyl-5-0-benzyl-isoidide.

In the subsequent course of the process, the compounds of the general formula IX in which $R^6$ and A have the meaning given are converted in a manner which is known per se into compounds of the general formula X in which $R^6$ and B have the meaning given, either by reacting them with nitrogen-containing heterocyclic bases of the general formula XI

$$B - H \qquad XI$$

in which B has the abovementioned meaning,

or by reacting them with protected derivatives of these nitrogen-containing heterocyclic bases, by which there are to be understood derivatives which are obtainable by reaction of the bases of the general formula XI with the protective groups customary in nucleoside chemistry, such as, for example, aliphatic or aromatic acyl radicals, in particular acetyl or benzoyl radicals,

or by reacting them with silylated derivatives of these nitrogen-containing heterocyclic bases, silylated derivatives being understood as those which are obtained by reaction of the bases of the general formula XI with trialkylsilyl halides, in particular trimethylsilyl chloride, or with hexaalkyl-disilazanes, in particular hexamethyl-disilazane, or with mixtures of the two. Such silylated derivatives can be prepared by methods with which the expert is familiar, and in this case without exception are known compounds, or by reacting them with metal salts of these nitrogen-containing heterocyclic bases, metal salts in general being understood as heavy metal salts, preferably silver and mercury salts; the metal salts of the bases can either be employed as such or advantageously formed in situ, this being carried out by adding stoichiometric or catalytic amounts of corresponding metal salts of inorganic or organic acids, in general heavy metal salts, preferably silver and mercury salts, such as, for example, silver carbonate, silver oxide, mercury chloride, mercury acetate, mercury bromide or mercury cyanide,

or by reacting them with alkoxy derivatives of these nitrogen-containing heterocyclic bases, "alkoxy" in general being understood as lower alkoxy groups, in particular methoxy and ethoxy groups.

In all these cases, the reactions can be carried out either with stoichiometric amounts or with an excess of one reactant and if appropriate in the presence of a suitable solvent for the reaction. Examples of possible solvents are: methylene chloride, dichloromethane, dichloroethane, chloroform, trichloroethane, acetonitrile, dioxane, tetrahydrofuran, dimethylformamine, sulfolane, benzene, toluene, xylene, chlorobenzene, carbon disulfide, carbon tetrachloride and nitromethane. If appropriate, the reaction with the silylated bases can also be carried out in the presence of a Lewis acid. Examples of suitable acids for this are silicon tetrachloride, tin tetrachloride, titanium tetrachloride, zinc chloride and boron trifluoride. It may be beneficial to add a tertiary amine for the reaction with the free heterocyclic bases. Possible tertiary amines are: tri-

lower alkylamines, such as trimethylamine, triethylamine and triisopropylamine, Hunig base or unsubstituted and substituted heterocyclic amines, such as pyridine, 4-dimethylaminopyridine, quinoline and the like. The reaction times and reaction temperatures are chosen according to the particular method and can vary within wide limits.

Quite generally, all methods which allow linkage of nucleoside bonds can be used for the conversion of IX into X. Sufficient methods and variants of this type are available and are familiar to any expert. For example, some of these processes are mentioned in the literature under the following names: Hilbert-Johnson reaction, Koenigs-Knorr synthesis, silylation process, Friedel-Crafts catalysed silyl method, melt process and corresponding variants of the above methods.

Review articles in this context are to be found, inter alia, in Basic Principles in Nucleic Acid Chemistry, Volume I, page 95 et seq., Academic Press, 1974, Nucleoside Analogues, page 35 et seq., Plenum Press, 1979, and Rodd's Chemistry of Carbon Compounds, Volume IV, L, page 125 et seq., Elsevier 1980.

The compounds of the general formula X obtained in this manner in which $R^6$ and B have the meaning given already represent some of the compounds of the general formula I according to the invention, in particular those in which R has all the other meanings given apart from hydrogen and a phosphate radical. They can be isolated and purified in the customary manner. If desired, they can subsequently be converted into compounds of the general formula I and if appropriate into salts thereof with inorganic and organic acids, preferably with pharmaceutically acceptable acids.

In the case where R in the general formula I denotes hydrogen, this conversion is carried out by splitting off the protective group $R^6$. If $R^6$ represents an aliphatic or aromatic acyl radical, this can be removed by the known methods of nucleoside protective group chemistry, such as, for example, by transesterification with ammonia/methanol, sodium methylate or potassium methylate/methanol or basic ion exchanger/methanol. If $R^6$ represents a benzyl radical, this can likewise be split off by known methods, for example by catalytic hydrogenation or catalysed transfer hydrogenation, or by treatment with boron tribromide or trimethylsilyl iodide.

Compounds of the general formula I in which R denotes a phosphate radical can be prepared in a manner which is known per se by reacting compounds of the general formula I in which R represents hydrogen with customary phosphorylating reagents, such as, for example, phosphorus oxytrichloride.

The salts which likewise belong to the subject matter of the invention are to be understood both as those with inorganic and those with organic acids, but preferably those with pharmaceutically acceptable acids. Examples of these are hydrochlorides, hydrobromides, nitrates, sulfates, phosphates, acetates, oxalates, fumarates, malates, maleates, malonates, tartrates, lactates, citrates, salicylates, methanesulfonates, benzenesulfonates, toluenesulfonates and naphthalenesulfonates.

These and other salts of the novel compounds, such as, for example, picrates, can if appropriate also be used for purification of the free bases by converting the free base into a salt, separating this off and if appropriate recrystallizing it or purifying it in another way, and liberating the base again from the purified salt.

The compounds of the general formula I according to the invention are distinguished by a pharmacological action profile such as has previously been shown by none of the isohexide derivatives already described. They have cytostatic, antiviral, enzyme-inhibiting and immuno-stimulating properties, coupled with a low toxicity, and can therefore be used, inter alia, as cytostatics, virustatics and immunostimulants. The present invention therefore also relates to the use of the compounds of the general formula I described here as medicaments, in particular for the prophylaxis and therapy of cancer and virus diseases and for eliminating disturbances in the immune system.

The compounds according to the invention can be administered in the customary manner for this purpose. Suitable presentation forms include, for example, oral, rectal, nasal, topical (including buccal, sublingual and ophthalmological), vaginal, parenteral (including subcutaneous, intramuscular, intravenous, intradermal, intrathecal and epidural) and transdermal administrations.

The medicaments administered can contain the compounds according to the invention in a content of 0.1 to 99.9%. The dosage depends on the envisaged intended purpose, the mode of administration, the severity of the illness and the evaluation of the treating physician. It is usually in the range from 0.1 to 300 mg once or several times daily per kg of body weight, preferably 1 to 50 mg per kg of body weight, it being possible to administer either single doses or multiple doses. In practice, unit doses of 1 to 250 mg once or several times daily are probably appropriate.

Possible pharmaceutical presentation forms are all the formulations with which the expert is familiar for the abovementioned administration, such as, for example, powders, granules, tablets, capsules, suppositories, suspensions, liquids, injectable preparations and transdermal systems. Solid, semi-solid or liquid excipients or diluents can be used to prepare these pharmaceutical presentation forms. These excipients

and diluents include correctants, binders, lubricants, emulsifiers and the like. Examples of such agents are starch, such as potato starch and cereal starch, sugars such as lactose, sucrose, glucose, mannitrol and sorbitol, cellulose, such as crystalline cellulose, methyl-cellulose, calcium carboxymethyl-cellulose, sodium carboxymethyl -cellulose and hydroxypropyl-cellulose, inorganic materials, such as potassium phosphate, calcium sulfate, calcium carbonate and talc, gelatin, gum arabic, polyvinylpyrrolidone, surface-active substances, such as fatty acid glycerides and fatty acid sorbitan esters, fatty acid esters of sucrose, polyglycerol and others.

Some examples of medicament formulations using the compounds of the general formula I according to the invention are shown below:

Tablets:

| | |
|---|---|
| Compound according to the invention | 100 mg |
| Lactose | 200 mg |
| Starch | 50 mg |
| Polyvinylpyrrolidone | 5 mg |
| Magnesium stearate | 4 mg |

Ointment:

| | |
|---|---|
| Compound according to the invention | 0.5 g |
| Glycerol | 15.0 g |
| Macrogol 300 | 20.0 g |
| Polyethylene glycol 1500 | 64.5 g |

The following examples serve to illustrate the invention.

The abbreviations in the examples have the following meanings:

MW = molecular weight
m.p. = melting point (uncorrected)
decomp. = decomposition
$[\alpha]_D^{20}$ = optical rotation at 20°C, sodium D line.

The optical rotation values are followed, in parentheses, by the concentrations of the solutions measured (for example c = 1 means 1 g/100 ml of solution) and the particular solvent.

## Example 1

2-0-(Uracil-1-yl-methyl)-isosorbide

a. 2-0-Chloromethyl-5-0-(4-toluoyl)-isosorbide

A suspension of 66 g of 5-0-(4-toluyl)-isosorbide (European Patent 67,964) and 15 of paraformaldehyde in 120 ml of methylene chloride is saturated with hydrogen chloride at 0° C, with cooling, a solution being obtained. The solution is left to stand at 0 to 5° C for 15-20 hours, the water which has separated out is removed and the solution is dried over calcium chloride. After filtration and concentration, an oil is obtained, which crystallizes when triturated. Crude yield 85 g. Recrystallization from methylene chloride/n-hexane gives an analytically pure product of m.p. 85-87° C; $[\alpha]_D^{20}$ + 63 (c = 2, methylene chloride).
$C_{15}H_{17}ClO_5$ MW 312.76

b. 2-0-(Uracil-1-yl-methyl)-5-0-(4-toluoyl)-isosorbide

12.8 g of 2,4-bis-(trimethylsilyl)-uracil (prepared by boiling uracil with excess hexamethyldisilazane until the evolution of ammonia has ended and subsequent distillation in vacuo) and 16 g of crude 2-0-chloromethyl-5-0-(4-toluoyl)-isosorbide are dissolved in 50 ml of anhydrous chloroform and the solution is stirred at room temperature until a complete reaction is recognizable in the thin layer chromatogram (about 2 hours). The reaction mixture is concentrated in vacuo as far as possible and the syrupy residue is dissolved in 100 ml of methylene chloride. 100 ml of saturated aqueous sodium bicarbonate solution are added and the mixture is stirred vigorously until the evolution of gas has ended. The organic phase is separated off, washed with water and dried over magnesium sulfate. Filtration and concentration of the filtrate gives a syrup which, when recrystallized from 50 ml of methanol, produces 12.7 g of product of m.p. 134 °C; $[\alpha]_D^{20}$ + 59.8 (c = 2, methylene chloride).
$C_{19}H_{20}N_2O_7$ MW 388.39

c. 2-0-(Uracil-1-yl-methyl)-isosorbide

11.65 g of 2-0-(uracil-1-yl-methyl)-5-0-(4-toluoyl)-isosorbide are suspended in 160 ml of methanol. 9 ml of 30% strength methanolic sodium methylate solution are added and the mixture is stirred at room temperature until a thin layer chromatogram indicates complete reaction (about 2 hours). The solution is now neutralized by addition of 90 ml of Amberlite IR-120 ($H^+$, methanol-washed), the ion exchanger is filtered off with suction and the filtrate is concentrated in vacuo. The residue is dissolved in 50 ml of methanol under the influence of heat, and on cooling the solution, crystals are obtained and are filtered off with suction, washed with methanol and ether and dried in vacuo.
Yield: 6.1 g; m.p. 161 °C; $[\alpha]_D^{20}$ + 41 (c = 1, water).
$C_{11}H_{14}N_2O_6$ MW 270.25

Example 2

2-0-(5-Fluorouracil-1-yl-methyl)-isosorbide

a. 2-0-(5-Fluorouracil-1-yl-methyl)-5-0-(4-toluyl)-isosorbide

13.7 g of 2,4-bis-(trimethylsilyl)-5-fluorouracil (prepared from 5-fluorouracil and hexamethyldisilazane analogously to Example 1 b.) and 16.4 g of crude 2-0-chloromethyl-5-0-(4-toluoyl)-isosorbide (Example 1 a.) are stirred in 50 ml of dry chloroform at room temperature. As soon as a thin layer chromatogram indicates complete reaction (about 4-5 hours), the mixture is concentrated in vacuo as far as possible, the foam which remains is dissolved in 100 ml of methylene chloride and the solution is treated with sodium bicarbonate solution and water and dried over magnesium sulfate, as described in Example 1 b. After the drying agent has been filtered off with suction, the solution is concentrated again in vacuo and 18.3 g of crude product are obtained as a foam. This is further processed without purification.
$C_{19}H_{19}FN_2O_7$ MW 406.38

b. 2-0-(5-Fluorouracil-1-yl-methyl)-isosorbide

A mixture of 18.3 g of crude 2-0-(5-fluorouracil-1-yl-methyl)-5-0-(4-toluoyl)-isosorbide, 240 ml of methanol and 13.5 ml of 30% strength sodium methylate solution is stirred at room temperature. When the reaction has ended (according to the thin layer chromatogram, after about 1 hour), the mixture is neutralized with 135 ml of methanol-moist Amberlite IR-120 ($H^+$) and filtered and the filtrate is evaporated in vacuo. The vitreous residue is purified by chromatography on silica gel (mobile phase ethyl acetate/methanol 9:1). A foam is obtained and is dissolved in water and lyophilized.
Yield: 3 g; m.p. 133-135 °C; $[\alpha]_D^{20}$ + 38 (c = 1, water).
$C_{11}F_{13}FN_2O_6$ MW 288.23

Example 3

2-0-(5-Bromouracil-1-yl-methyl)-isosorbide

a. 2-0-(5-Bromouracil-1-yl-methyl)-5-0-(4-toluyl)-isosorbide

16.8 g of 2,4-bis-(trimethylsilyl)-5-bromouracil (synthesized in accordance with Example 1 b. from 5-bromouracil and hexamethyldisilazane) and 16 g of crude 2-0-chloromethyl-5-0-(4-toluoyl)-isosorbide (Example 1 a.) are dissolved in 50 ml of dry chloroform and the solution is stirred at room temperature until a complete reaction is recognizable in the thin layer chromatogram (about 20 hours). After concentration in vacuo, dissolving of the residue in 100 ml of methylene chloride, stirring of the solution with sodium bicarbonate solution for one hour and washing with water, a clear solution is obtained which is dried over magnesium sulfate, filtered and concentrated in vacuo. The foam thus obtained (27.8 g) of the crude title compound is further reacted directly.
$C_{19}H_{19}BrN_{2}0_{7}$MW 467.30

b. 2-0-(5-Bromouracil-1-yl-methyl)-isosorbide

15 ml of 30% strength sodium methylate solution are added to 27.8 g of crude 2-O-(5-bromouracil-1-yl-methyl)-5-0-(4-toluoyl)-isosorbide and 270 ml of methanol and the mixture is stirred at room temperature until a thin layer chromatogram indicates complete transesterification (about 1 hour). The mixture is neutralized with 120 ml of Amberlite IR-120 ($H^+$, methanol-washed) and filtered with suction and the filtrate is concentrated in vacuo. The syrup which remains is dissolved in 150 ml of methanol and the solution is cooled to 0°. After a short time, a precipitate separates out. The precipitate is filtered off with suction and recrystallized from 95% pure aqueous methanol. Yield: 17 g; m.p. 169° C; $[\alpha]_D^{20}$ + 35.5 (c = 1, water).
$C_{11}H_{13}BrN_{2}0_{6}$MW 349.16

Example 4

2-0-(5-Iodouracil-1-yl-methyl)-isosorbide

a. 2-0-(5-Iodouracil-1-yl-methyl)-5-0-(4-toluyl)-isosorbide

19.1 g of 2,4-bis-(trime hylsilyl)-5-iodouracil (prepared analogously to Example 1 b. from 5-iodouracil and hexamethyldisilazane) and 16.4 g of 2-0-chloromethyl-5-0-(4-toluoyl)-isosorbide (Example 1 a.) are dissolved in 50 ml of dry chloroform and the solution is left to stand at room temperature until the reaction is complete (according to the thin layer chromatogram, about 20 hours). All the volatile portions are evaporated off in vacuo, the vitreous residue is dissolved in 100 ml of acetone, the solution is clarified with active charcoal and the resulting solution is slowly added dropwise to 1.5 liters of water. The amorphous precipitate which has separated out is filtered off with suction, washed with water and dried at 40° in vacuo. Yield: 23.8 g of crude product, which is used in this form in the next stage.
$C_{19}H_{19}IN_{2}0_{7}$MW 514.29

b. 2-0-(5-Iodouracil-1-yl-methyl)-isosorbide

15.4 g of 2-0-(5-iodouracil-1-yl-methyl)-5-0-(4-toluoyl)-isosorbide in 160 ml of methanol are stirred with 9 ml of 30% strength sodium methylate solution until a thin layer chromatogram indicates a complete reaction (about 2 hours). After neutralization with 90 ml of methanol-washed Amberlite IR-120 ($H^+$), the exchanger is filtered off with suction, the filtrate is evaporated in vacuo and the semi-solid residue is stirred with methanol and ether. The solid is filtered off with suction and recrystallized from methanol with active charcoal.
Yield: 5.8 g; m.p. 148-149° C; $[\alpha]_D^{20}$ + 30 (c = 1, water).

$C_{11}H_{13}IN_2O_6$ MW 396.15

Example 5

2-0-(5-Methyluracil-1-yl-methyl)-isosorbide

Method 1

13.5 g of 2,4-bis-(trimethylsilyl)-5-methyluracil (preparation from 5-methyluracil and hexamethyldisilazane analogously to Example 1 b.) are dissolved in 50 ml of dry chloroform, and 16.5 g of crude 2-0-chloromethyl-5-0-(4-toluoyl)-isosorbide (Example 1 a.) are added. The mixture is stirred until a clear solution is obtained and is left to stand until the reaction is complete (about 4 hours, according to the thin layer chromatogram). Thereafter, it is concentrated in vacuo, the syrup of crude 2-0-(5-methyluracil-1-yl-methyl)-5-0-(4-toluoyl)-isosorbide is dissolved in 100 ml of methanol, the solution is concentrated again and the residue is dissolved in 275 ml of methanol. After addition of 15 ml of 30% strength sodium methylate solution, the mixture is stirred at room temperature until, according to the thin layer chromatogram, the transesterification is complete (about 2 hours). The mixture is neutralized in the customary manner by addition of 120 ml of Amberlite IR-120 ($H^+$, methanol-moist) and is filtered and the filtrate is concentrated in vacuo. The oily product is stirred with diethyl ether, this is decanted off and the syrupy residue is dissolved in 30 ml of warm ethanol. The solution is cooled gradually to 0° C and the precipitate which has separated out is filtered off with suction and recrystallized from methanol. Yield: 6 g; m.p. 132-133° C; $[\alpha]_D^{20}$ + 39 (c = 1, water).
$C_{12}H_{16}N_2O_6$ MW 284.27

Method 2

a. 2-0-Chloromethyl-5-0-(2-nitrobenzoyl)-isosorbide

100 g of 5-0-(2-nitrobenzoyl)-isosorbide [Starch/Stärke 36, 170 (1984)] and 20.4 g of paraformaldehyde are suspended in 200 ml of methylene chloride, and hydrogen chloride is passed in at 0° C until the suspension is saturated. After the mixture has been left to stand at 0 to 2° C for four days, the water which has separated out is removed and the organic phase is dried over calcium chloride and concentrated in vacuo as far as possible. The syrupy crude product is further processed in this form. Yield: 125 g.
$C_{14}H_{14}ClNO_7$ MW 343.73

b. 2-0-(5-Methyluracil-1-yl-methyl)-5-0-(2-nitrobenzoyl)-isosorbide

13.5 g of 2,4-bis-(trimethylsilyl)-5-methyluracil (prepared analogously to Example 1 b. from 5-methyluracil) and 19 g of crude 2-0-chloromethyl-5-o-(2-nitrobenzoyl)-isosorbide are stirred in 50 ml of anhydrous chloroform at room temperature until a thin layer chromatogram reveals that the reaction is complete (5 hours). After all the volatile portions have been distilled off in vacuo, the residue is dissolved in 100 ml of methylene chloride, the solution is stirred with 100 ml of saturated sodium bicarbonate solution for 1-2 hours, the organic phase is separated off, dried over magnesium sulfate, filtered and evaporated and the residue is recrystallized from ethanol. Yield: 17 g; m.p. 169-170° C; $[\alpha]_D^{20}$ + 30.3 (c = 2, methylene chloride).
$C_{19}H_{19}N_3O_9$ MW 433.39

c. 2-0-(5-Methyluracil-1-yl-methyl)-isosorbide

15 g of 2-0-(5-methyluracil-1-yl-methyl)-5-0-(2-nitrobenzoyl)-isosorbide, suspended in 150 ml of methanol, are reacted with 10 ml of 30% strength sodium methylate solution in the customary manner (duration of

1 hour, according to the thin layer chromatogram). After neutralization with 100 ml of Amberlite IR-120 ($H^+$, methanol-washed) and filtration, the filtrate is evaporated in vacuo and the semi-solid residue is recrystallized from methanol. Yield: 5.5 g; physical data identical to those of the product prepared according to method 1.

Method 3

a. 5-0-(4-Chlorobenzoyl)-isosorbide

270 g of 4-chlorobenzoyl chloride are added dropwise to a solution of 146 g of isosorbide in 1 l of pyridine at 15-20 ° C, with cooling. Stirring is then continued at 20-25 ° C until, according to the thin layer chromatogram, the reaction has ended. The volatile constituents of the mixture are evaporated off in vacuo as far as possible, the residue is taken up in 1 l of methylene chloride and this solution is extracted by shaking in succession with in each case 1 l of 2 N sulfuric acid, saturated sodium bicarbonate solution and water. Drying over magnesium sulfate, filtration and concentration in vacuo gives a solid residue, which is stirred with ether and filtered off with suction. The crude product is purified by recrystallization from toluene. Yield: 70 g; m.p. 155-156 ° C; $[\alpha]_D^{20}$ + 34.5 (c = 1, methylene chloride).
$C_{13}H_{13}ClO_5$ MW 284.70

b. 5-0-(4-Chlorobenzoyl)-2-0-chloromethyl-isosorbide

A suspension of 68.4 g of 5-0-(4-chlorobenzoyl)-isosorbide and 15.2 g of paraformaldehyde in 150 ml of methylene chloride is saturated with hydrogen chloride at 0 ° , a solution being obtained. The solution is left to stand at 0 to 2 ° C for 15-20 hours, the water which has separated out is removed, the mixture is dried over calcium chloride and, after filtration, the filtrate is concentrated in vacuo. The syrupy crude product crystallizes on trituration. Yield: 78.8 g. The compound can be obtained in a pure form by recrystallization from methylene chloride/n-hexane. m.p. 66-69 ° C; $[\alpha]_D^{20}$ + 35 (c = 2, methylene chloride).
$C_{14}H_{14}Cl_2O_5$ MW 333.18

c. 5-0-(4-Chlorobenzoyl)-2-0-(5-methyluracil-1-yl-methyl)-isosorbide

19.8 g of 2,4-bis-(trimethylsilyl)-5-methyluracil (preparation from 5-methyluracil analogously to Example 1 b.) and 24.5 g of crude 5-0-(4-chlorobenzoyl)-2-0-chloromethyl-isosorbide are dissolved in 100 ml of chloroform and the solution is left to stand at room temperature until the reaction is complete (thin layer chromatography check, 18 hours). The solution is treated as described in method 2 under b. and the foamy crude product is purified by chromatography on silica gel (mobile phase chloroform/methanol 9:1). The reaction product is obtained as a white amorphous substance. Yield: 22.4 g; m.p. 75-80 ° C; $[\alpha]_D^{20}$ + 51 (c = 2, methylene chloride).
$C_{19}H_{19}ClN_2O_7$ MW 422.83

d. 2-0-(5-Methyluracil-1-yl-methyl)-isosorbide

2-0-(5-Methyluracil-1-yl-methyl)-isosorbide

11.2 g of 5-0-(4-chlorobenzoyl)-2-0-(5-methyluracil-1-yl-methyl)-isosorbide and 10 ml of 30% strength sodium methylate solution are stirred in 100 ml of methanol for 1 hour and the mixture is worked up as described in method 2 under c. After recrystallization from methanol, 5.6 g of product are isolated; the physical data are identical to those of the substance obtained by method 1.

Example 6

2-0-(5-Ethyluracil-1-yl-methyl)-isosorbide

a. 2-0-(5-Ethyluracil-1-yl-methyl)-5-0-(4-toluoyl)-isosorbide

14.2 g of 2,4-bis-(trimethylsilyl)-5-ethyluracil (synthesized from 5-ethyluracil according to Example 1 b.) and 15.6 g of crude 2-0-chloromethyl-5-0-(4-toluoyl)-isosorbide (Example 1 a.) are dissolved in 50 ml of dry chloroform and the solution is allowed to react at room temperature. After 3.5 hours, a thin layer chromatogram indicates complete reaction. The mixture is concentrated in vacuo as far as possible, the foam which remains is dissolved in 100 ml of methylene chloride and this solution is stirred vigorously with 100 ml of saturated sodium bicarbonate solution for 60 minutes. The organic phase is separated off, rinsed with water and dried over magnesium sulfate and, after filtration, the filtrate is concentrated in vacuo. The vitreous residue is dissolved in methanol and the solution is left to stand at room temperature for several hours, crystallization occurring. The crystals are filtered off with suction and rinsed with methanol. Yield: 13.45 g; m.p. 134° C; $[\alpha]_D^{20}$ + 68 (c = 2, methylene chloride).
$C_{21}H_{24}N_2O_7$ MW 416.44

b. 2-0-(5-Ethyluracil-1-yl-methyl)-isosorbide

12.5 g of 2-0-(5-ethyluracil-1-yl-methyl)-5-0-(4- toluoyl)-isosorbide are dissolved in 160 ml of methanol and, after addition of 9 ml of 30% strength sodium methylate solution, the mixture is stirred at room temperature until a thin layer chromatogram indicates that the toluoyl radical has been completely split off (2 hours). The mixture is neutralized in the customary manner with 70 ml of methanol-washed Amberlite IR-120 ($H^+$) and filtered and the filtrate is concentrated in vacuo. The oily residue is dissolved in 50 ml of water and the solution is extracted several times with ether until a thin layer chromatogram indicates the absence of methyl 4-toluylate. A further 50 ml of water are added and the solution is subjected to freeze-drying, 6 g of product being obtained. m.p. 48-60° C; $[\alpha]_D^{20}$ + 38 (c = 1, water).
$C_{13}H_{18}N_2O_6$ . 0.5 $H_2O$ MW 307.31

Example 7

2-0-(5-Isobutyluracil-1-yl-methyl)-isosorbide

a. 2-0-(5-Isobutyluracil-1-yl-methyl)-5-0-(4-toluoyl)isosorbide

15.6 g of crude 2,4-bis-(trimethylsilyl)-5-isobutyluracil (prepared by boiling 8.4 g of 5-isobutyluracil with excess hexamethyldisilazane with the addition of catalytic amounts of ammonium sulfate until the evolution of ammonia has ended, distilling off the excess hexamethyldisilazane and degassing the mixture at 90° C in vacuo for 1-2 hours) and 16.5 g of crude 2-0-chloromethyl-5-0-(4-toluoyl)-isosorbide (Example 1 a.) are dissolved in 50 ml of anhydrous chloroform and the solution is left to react at room temperature until, according to the thin layer chromatogram, everything has reacted (about 20 hours). All the volatile portions are now distilled off in vacuo, the syrupy residue is taken up in 100 ml of methylene chloride and the solution is stirred with 100 ml of saturated sodium bicarbonate solution until no further carbon dioxide is evolved. The organic phase is separated off, washed with water and concentrated in vacuo. The vitreous residue is recrystallized from 70% strength aqueous methanol. Yield: 17.3 g; m.p. 169-170° C; $[\alpha]_D^{20}$ + 45.3 (c = 2, methylene chloride).
$C_{23}H_{28}N_2O_7$ MW 444.49

b. 2-0-(5-Isobutyluracil-1-yl-methyl)-isosorbide

15 g of 2-0-(isobutyluracil-1-yl-methyl)-5-0-(4-toluoyl)-isosorbide are suspended in 125 ml of methanol. After addition of 10 ml of 30% strength sodium methylate solution, the mixture is stirred at room temperature until complete esterification is recognizable in the thin layer chromatogram (1 hour). 80 ml of

Amberlite IR-120 ($H^+$, methanol-washed) are added, the mixture is stirred until moist indicator paper inditates a neutral reaction, the exchanger is filtered off and the filtrate is concentrated in vacuo to a large degree. The oily residue is stirred thoroughly with diethyl ether, this is decanted off and the product is purified on a column (silica gel, mobile phase chloroform/methanol 9:1). The fractions containing the desired compound are evaporated, the residue is taken up in water and the mixture is freeze-dried. 6 g of an amorphous powder are obtained; m.p. 53-62° C; $[\alpha]_D^{20}$ + 34.5 (c = 1, water).

$C_{15}H_{22}N_2O_6$ . 0.25 $H_2O$MW 330.86

Example 8

2-0-(5-n-Hexyluracil-1-yl-methyl)-isosorbide

a. 2-0-(5-n-Hexyluracil-1-yl-methyl)-5-0-(4-toluyl)-isosorbide

17 g of crude 2,4-bis-(trimethylsilyl)-5-n-hexyluracil (synthesized analogously to Example 7 a. from 9.8 g of 5-n-hexyluracil) are dissolved in 50 ml of dry chloroform, and 16.5 g of crude 2-0-chloromethyl-5-0-(4-toluoyl)-isosorbide (Example 1 a.) are added. The end of the reaction is detected by thin layer chromatography (about 1 hour). The mixture is concentrated in vacuo as far as possible and the vitreous residue is dissolved in diethyl ether. After the solution has been left to stand in a refrigerator overnight, the product which has precipitated is filtered off with suction and washed with diethyl ether. Yield: 19 g; m.p. 104-106° C; $[\alpha]_D^{20}$ + 48 (c = 2, methylene chloride).

$C_{25}H_{32}N_2O_7$MW 472.55

b. 2-0-(5-n-Hexyluracil-1-yl-methyl)-isosorbide

18.5 g of 2-0-(5-n-hexyluracil-1-yl-methyl)-5-0-(4-toluoyl)-isosorbide, 100 ml of methanol and 12.5 ml of 30% strength sodium methylate solution are stirred at room temperature until a thin layer chromatogram indicates complete reaction (1 hour). The mixture is neutralized by addition of 100 ml of Amberlite IR-120 ($H^+$, methanol-washed), the exchanger is filtered off and the filtrate is concentrated in vacuo. The oily residue is purified by chromatography on silica gel (mobile phase ethyl acetate). Concentration of the corresponding fractions gives 3.5 g of the pure title compound as a waxy product; m.p. about 50-55° C; $[\alpha]_D^{20}$ + 29.5 (c = 2, methylene chloride).

$C_{17}H_{26}N_2O_6$ . 0.5 $H_2O$MW 363.42

Example 9

2-0-(5-Hydroxymethyluracil-1-yl-methyl)-isosorbide

a. 2-0-(5-Hydroxymethyluracil-1-yl-methyl)-5-0-(4-toluoyl)-isosorbide

18 g of 2,4-bis-(trimethylsilyl)-hydroxymethyluracil (prepared analogously to Example 1 b. from 5-hydroxymethyluracil) and 16 g of crude 2-0-chloromethyl-5-0-(4-toluoyl)-isosorbide (Example 1 a.) are dissolved in 50 ml of anhydrous chloroform and the solution is left to stand at room temperature until the reaction is complete (2 hours, according to the thin layer chromatogram). The mixture is concentrated to a syrup and this is dissolved in 75 ml of methanol, crystallization occurring. The crystals are filtered off with suction and the crude product is recrystallized from 80% strength aqueous methanol.

Yield: 13 g; m.p. 192-193° C; $[\alpha]$ + 49.3 (c = 2, dimethylformamide).

$C_{20}H_{22}N_2O_8$MW 418.41

b. 2-0-(5-Hydroxymethyluracil-1-yl-methyl)-isosorbide

16.7 g of 2-0-(5-hydroxymethyluracil-1-yl-methyl)-5-0-(4-toluoyl)-isosorbide are dissolved in 220 ml of methanol, 12 ml of 30% strength sodium methylate solution are added and the mixture is stirred until a thin layer chromatogram indicates complete transesterification (2.5 hours). After neutralization with 120 ml of Amberlite IR-120 ($H^+$, methanol-moist), the mixture is heated to the boiling point, the exchanger is filtered off hot with suction and rinsed with 150 ml of hot methanol and the combined filtrates are cooled to 0 to 5° C. The solid which has precipitated is filtered off with suction and recrystallized from 80% strength aqueous methanol.
Yield: 5.7 g; m.p. 177-178° C; $[\alpha]_D^{20}$ + 36.5 (c = 1, water).
$C_{12}H_{16}N_2O_7$ MW 300.27

Example 10

2-0-(5-Trifluoromethyluracil-1-yl-methyl)-isosorbide

16.5 g of crude 2-0-chloromethyl-5-0-(4-toluoyl)-isosorbide (Example 1 a.) are added to 16.2 g of 2,4-bis-(trimethylsilyl)-5-trifluoromethyluracil (preparation analogously to Example 1 b. from 5-trifluoromethyluracil), dissolved in 50 ml of dried chloroform, and the mixture is allowed to react at room temperature until a complete reaction is recognizable in the thin layer chromatogram (48 hours). The mixture is concentrated in vacuo, the vitreous residue is taken up in 100 ml of methylene chloride, this solution is stirred with 100 ml of saturated sodium bicarbonate solution for 2 hours and the organic layer is separated off and dried over magnesium sulfate. The organic layer is filtered and evaporated in vacuo and the residue of crude 2-0-(5-trifluoromethyluracil-1-yl-methyl)-5-0-(4-toluoyl)-isosorbide is dissolved in 225 ml of methanol. After addition of 15 ml of 30% strength sodium methylate solution, the mixture is stirred at room temperature until, according to the thin layer chromatogram, the transesterification is complete, the mixture is neutralized with 200 ml of Amberlite IR-120 ($H^+$, methanol-washed) and filtered and the filtrate is concentrated in vacuo. The crude substance is purified by chromatography on silica gel (mobile phase ethyl acetate). The fractions containing the desired product are combined and concentrated. The crystalline residue is analytically pure. Yield: 4.8 g; m.p. 155-158° C; $[\alpha]_D^{20}$ + 55 (c = 1, methanol).
$C_{12}H_{13}F_3N_2O_6$ MW 338.25

Example 11

2-0-[E-5-(2-Bromovinyl)-uracil-1-yl-methyl]-isosorbide

a. 2-0-[E-5-(2-Bromovinyl)-uracil-1-yl-methyl]-5-0-(4-toluoyl)-isosorbide

18.1 g of crude 2,4-bis-(trimethylsilyl)-[E-5-(2-bromovinyl)-uracil] (prepared analogously to Example 7 a. from 10.85 g of E-5-(2-bromovinyl)-uracil) and 16.5 g of crude 2-0-chloromethyl-5-0-(4-toluoyl)-isosorbide (Example 1 a.) are dissolved in 50 ml of dry chloroform and the course of the reaction is monitored by means of thin layer chromatography. As soon as the reaction is complete (about 7 hours) the solvent is evaporated off in vacuo and the syrupy residue is stirred with 50 ml of methanol. After some time, crystallization thereby occurs. The crystals are filtered off with suction and the crude product is recrystallized from methanol.
Yield: 14 g; m.p. 117-120° C; $[\alpha]$ + 42 (c = 1, methylene chloride).
$C_{21}H_{21}BrN_2O_7$ MW 493.33

b. 2-0-[E-5-(2-Bromovinyl)-uracil-1-yl-methyl]-isosorbide

14 g of 2-0-[E-5-(2-bromovinyl)-uracil-1-yl-methyl]-5-0-(4-toluoyl)-isosorbide are dissolved in 200 ml of methanol, 9 ml of 30% strength sodium methylate solution are added and the mixture is stirred until the thin layer chromatogram shows that the protective group has been split off completely (1 hour). The mixture is neutralized with 90 ml of methanol-washed Amberlite IR-120 ($H^+$) in the customary manner, the ion

exchanger is filtered off and the filtrate is concentrated in vacuo as far as possible. The partly crystalline residue is stirred with diethyl ether until everything has become powdery and the powder is then filtered off with suction. The crude product is recrystallized from methanol.
Yield: 4.6 g; m.p. 141-141.5° C; $[\alpha]_D^{20}$ + 34.5 (c = 0.33, water).
$C_{13}H_{15}BrN_2O_6$ MW 375.19

Example 12

2-0-(Cytosin-1-yl-methyl)-isosorbide

a. 2-0-(Cytosin-1-yl-methyl)-5-O-(4-toluoyl)-isosorbide

12.8 g of crude 2,4-bis-(trimethylsilyl)-cytosine (synthesized analogously to Example 7 a. from 5.6 g of cytosine) are dissolved in 50 ml of dried chloroform, and 16 g of crude 2-0-chloromethyl-5-0-(4-toluyl)-isosorbide (Example 1 a.) are added. As soon as a complete reaction is recognizable in the thin layer chromatogram (3 hours) the mixture is concentrated in vacuo, the residue is dissolved in 100 ml of methylene chloride, the solution is stirred with 100 ml of saturated sodium bicarbonate solution for 1 hour, the organic phase is separated off and concentrated and the solid thus obtained is stirred with water. The solid is filtered off with suction and dried in vacuo at 40°. The crude product is recrystallized from 80% strength aqueous methanol. Yield: 8.5 g; m.p. 214-215° C (decomp.); $[\alpha]_D^{20}$ + 68.5 (c = 2, dimethylformamide).
$C_{19}H_{21}N_3O_6$ MW 387.40

b. 2-0-(Cytosin-1-yl-methyl)-isosorbide

12 ml of 30% strength sodium methylate solution are added to 15.5 g of 2-0-(cytosin-1-yl-methyl)-5-0-(4-toluoyl)-isosorbide, suspended in 220 ml of methanol. After transient solution, a precipitate soon separates out again. After 30 minutes, a thin layer chromatogram indicates complete reaction. The mixture is neutralized by addition of 4 ml of glacial acetic acid, cooled to 0 to 5° C for 1 hour and filtered with suction and the residue is washed with ethanol and diethyl ether. The crude product is recrystallized from 80% strength aqueous methanol. Yield: 8 g; m.p. 255- 56° C (decomp.); $[\alpha]_D^{20}$ + 47.5 (c = 1, water)
$C_{11}H_{15}N_3O_5$ MW 269.26

Example 13

2-0-(5-Fluorocytosin-1-yl-methyl)-isosorbide

a. 2-0-(5-Fluorocytosin-1-yl-methyl)-5-0-(4-toluoyl)-isosorbide

13.7 g of crude 2,4-bis-(trimethylsilyl)-5- fluorocytosine (prepared from 6.5 g of 5-fluorocytosine analogously to Example 7 a.) and 16.5 g of crude 2-0-chloromethyl-5-0-(4-toluoyl)-isosorbide (Example 1 a.) are dissolved in 50 ml of dry chloroform. After a short time, a precipitate starts to separate out. When everything has reacted (after 20 hours, according to the thin layer chromatogram), all the volatile portions are evaporated off in vacuo and the residue is recrystallized from methanol. Yield: 12.4 g; m.p. 237.5-238° C; $[\alpha]$ + 56 (c = 2, dimethylformamide).
$C_{19}H_{20}FN_3O_6$ MW 405.39

b. 2-0-(5-Fluorocytosin-1-yl-methyl)-isosorbide

12.15 g of 2-0-(5-fluorocytosin-1-yl-methyl)-5-0-(4-toluoyl)-isosorbide and 9 ml of 30% strength sodium

methylate solution are stirred in 165 ml of methanol until a thin layer chromatogram indicates that complete reaction has taken place (45 minutes). The mixture is neutralized with 3 ml of glacial acetic acid and the solid which has precipitated is filtered off with suction and recrystallized from 80% strength methanol. Yield: 4.7 g; m.p. 250-251 °C (decomp.), $[\alpha]_D^{20}$ + 39.5 (c = 1, water).

$C_{11}H_{14}FN_3O_5$ MW 287.26

The hydrochloride is obtained by dissolving in methanolic hydrochloric acid and precipitating with diethyl ether. m.p. 189 °C (decomp.); $[\alpha]_D^{20}$ + 32 (c = 1, water).

$C_{11}H_{14}FN_3O_5$ . HCl MW 323.72

Example 14

2-0-(5-Chlorocytosin-1-yl-methyl)-isosorbide

a. 5-0-Benzoyl-2-0-chloromethyl-isosorbide

89.5 g of 5-0-benzoyl-isosorbide [Carbohydr. Res. 39, 358 (1975)] are dissolved in 165 ml of methylene chloride, 21.5 g of paraformaldehyde are added and the mixture is saturated with hydrogen chloride at 0 °C. The mixture is left to stand at 0 to 2 °C for 18-20 hours, the water of reaction which has separated out is removed, the methylene chloride solution is dried over calcium chloride and then over magnesium sulfate and filtered and the filtrate is concentrated in vacuo. The crude product obtained as a viscous oil is reacted further in this form. Yield: 112 g.

$C_{14}H_{15}ClO_5$ MW 298.73

b. 5-0-Benzoyl-2-0-(5-chlorocytosin-1-yl-methyl)-isosorbide

14.5 g of crude 2,4-bis-(trimethylsilyl)-5-chlorocytosine (prepared analogously to Example 7 a. from 7.3 g of 5-chlorocytosine) and 16 g of crude 5-0-benzoyl-2-0-chloromethyl-isosorbide are dissolved in 50 ml of chloroform (anhydrous) and the solution is stirred at room temperature until the reaction has ended (18 hours). The mixture is evaporated in vacuo, the syrupy residue is dissolved in 100 ml of methylene chloride, the solution is stirred with 100 ml of sodium bicarbonate solution for 1 hour and the organic layer is separated off and concentrated as far as possible. The crude product is purified by chromatography on silica gel (mobile phase chloroform/methanol 5:1). Yield: 4.6 g; m.p. 199-201 °C; $[\alpha]_D^{20}$ + 62 (c = 1, methylene chloride).

$C_{18}H_{18}ClN_3O_6$ MW 407.82

c. 2-0-(5-Chlorocytosin-1-yl-methyl)-isosorbide

9 g of 5-0-benzoyl-2-0-(5-chlorocytosin-1-yl-methyl)-isosorbide, suspended in 100 ml of methanol, are stirred with 4 ml of 30% strength sodium methylate solution at room temperature for 1 hour; a thin layer chromatogram shows that the benzoyl group has been split off completely. The mixture is neutralized by stirring with 40 ml of Amberlite IR-120 ($H^+$, methanol-washed) and filtered and the filtrate is concentrated to dryness in vacuo. The residue is recrystallized from ethanol. Yield: 3.4 g; m.p. 214 °C; $[\alpha]_D^{20}$ + 49.5 (c = 1, water).

$C_{11}H_{14}ClN_3O_5$ MW 303.71

Example 15

2-0-(5-Iodocytosin-1-yl-methyl)-isosorbide

a. 2-0-(5-Iodocytosin-1-yl-methyl)-5-0-(4-toluoyl)-isosorbide

19 g of crude 2,4-bis-(trimethylsilyl)-5-iodocytosine (synthesized analogously to Example 7 a. from 11.85 g of 5-iodocytosine) are dissolved in 50 ml of dry chloroform, 16 g of crude 2-0-chloromethyl-5-0-(4-toluoyl)-isosorbide (Example 1 a.) are added and the mixture is stirred at room temperature until a thin layer chromatogram indicates complete reaction (2 hours). The mixture is concentrated in vacuo, the vitreous residue is dissolved in 100 ml of methylene chloride and this solution is stirred with 100 ml of saturated sodium bicarbonate solution for 1 hour. The organic phase is separated off and concentrated in vacuo as far as possible. The residue is recrystallized from methanol. Yield: 9.1 g; m.p. 190-191 °C (decomp.); $[\alpha]_D^{20}$ + 42.5 (c = 2, methylene chloride).
$C_{19}H_{20}IN_3O_6$ MW 513.30

b. 2-0-(5-Iodocytosin-1-yl-methyl)-isosorbide

10.3 g of 2-0-(5-iodocytosin-1-yl-methyl)-5-0-(4-toluoyl)-isosorbide are suspended in 110 ml of methanol. 6 ml of 30% strength sodium methylate solution are added dropwise and the mixture is stirred until complete reaction can be seen in the thin layer chromatogram (30 minutes). The mixture is neutralized with 2 ml of glacial acetic acid and kept at 0 to 5 °C for 1 hour and the crude product which has precipitated is filtered off with suction and recrystallized from methanol with the addition of active charcoal. Yield: 4.3 g; m.p. 181 °C (decomp.); $[\alpha]_D^{20}$ + 32 (c = 1, water).
$C_{11}H_{14}IN_3O_5$ MW 395.17

Example 16

2-0-(Isocytosin-1-yl-methyl)-isosorbide

a. 2-0-(Isocytosin-1-yl-methyl)-5-0-(4-toluoyl)-isosorbide

12.8 g of crude 2,4-bis-(trimethylsilyl)-isocytosine (prepared analogously to Example 7 a. from 5.6 g of isocytosine) and 16 g of crude 2-0-chloromethyl-5-0-(4-toluoyl)-isosorbide (Example 1 a.), dissolved in 50 ml of dry chloroform, are stirred at room temperature until complete reaction can be seen from a thin layer chromatogram (2 hours). The mixture is evaporated in vacuo, the residue is dissolved in 100 ml of methylene chloride, the solution is stirred with 100 ml of sodium bicarbonate solution for 1 hour, the organic phase is separated off and concentrated as far as possible and the residue is recrystallized from methanol. Yield: 5.2 g; m.o. 223-225 °C (decomp.); $[\alpha]_D^{20}$ + 53.5 (c = 2, dimethylformamide).
$C_{19}H_{21}N_3O_6$ MW 387.40

b. 2-0-(Isocytosin-1-yl-methyl)-isosorbide

5.2 g of 2-0-(isocytosin-1-yl-methyl)-5-0-(4-toluoyl)-isosorbide and 4 ml of 30% strength sodium methylate solution are stirred in 70 ml of methanol until, according to the thin layer chromatogram, splitting off of the toluyl group has ended (1 hour). The mixture is neutralized by addition of 40 ml of methanol-washed ion exchanger (Amberlite IR-120, $H^+$), the exchanger is filtered off, the filtrate is evaporated in vacuo and the solid residue is recrystallized from ethanol. Yield: 2.3 g; m.p. 192-193 °C (decomp.); $[\alpha]_D^{20}$ + 45.5 (c = 1, water).
$C_{11}H_{15}N_3O_5$ MW 269.26

Example 17

2-0-(5-Methylisocytosin-1-yl-methyl)-isosorbide

a. 2-0-(5-Methylisocytosin-1-yl-methyl)-5-0-(4-toluoyl)-isosorbide

27 g of crude 2,4-bis-(trimethylsilyl)-5-methylisocytosine (prepared analogously to Example 7 a. from 12.5 g of 5-methylisocytosine) are dissolved in 100 ml of dry chloroform, and 33 g of crude 2-0-chloromethyl-5-0-(4-toluyl)-isosorbide (Example 1 a.) are added. The mixture is stirred until complete reaction can be seen in the thin layer chromatogram (15 hours) and is concentrated in vacuo and the oily residue is recrystallized from methanol. Yield: 18.3 g; m.p. 165-166 °C; $[\alpha]_D^{20}$ + 56.5 (c = 2, dimethylformamide).
$C_{20}H_{23}N_3O_6$ MW 401.43

b. 2-0-(5-Methylisocytosin-1-yl-methyl)-isosorbide

18.3 g of 2-0-(5-methylisocytosin-1-yl-methyl)-5-0-(4-toluoyl)-isosorbide, suspended in 200 ml of methanol, are stirred with 15 ml of 30% strength sodium methylate solution at room temperature until everything has dissolved and a thin layer chromatogram indicates complete reaction (1 hour). 150 ml of methanol-washed Amberlite IR-120 ($H^+$) are added, the mixture is stirred until the solution has a neutral reaction, the exchanger is filtered off with suction and the filtrate is evaporated in vacuo. The waxy residue is recrystallized from methanol. Yield: 6.2 g; m.p. 197-198 °C; $[\alpha]_D^{20}$ + 42.5 (c = 1, water).
$C_{12}H_{17}N_3O_5$ MW 283.29

Example 18

2-0-(3-Methoxycarbonyl-1,2,4-triazol-1-yl-methyl)-isosorbide

a. 2-0-(3-Methoxycarbonyl-1,2,4-triazol-1-yl-methyl)-5-0-(4-toluoyl)-isosorbide and 2-0-(5-methoxy-carbonyl-1,2,4-triazol-1-yl-methyl)-5-0-(4-toluyl)-isosorbide

20 g of the crude trimethylsilyl derivative of methyl 1,2,4-triazole-3-carboxylate (prepared analogously to Example 7 a. from 12.7 g of methyl 1,2,4-triazole-3-carboxylate) and 33 g of crude 2-0-chloromethyl-5-0-(4-toluoyl)-isosorbide (Example 1 a.) are dissolved in 100 ml of dry chloroform and the solution is allowed to react at room temperature until, according to the thin layer chromatogram, the reaction has ended (20 hours). The mixture is evaporated in vacuo, the vitreous residue is dissolved in 200 ml of methylene chloride, the solution is stirred with 100 ml of sodium bicarbonate solution for 1 hour and the methylene chloride layer is separated off, dried over magnesium sulfate and concentrated in vacuo. The residue is purified by chromatography on silica gel (mobile phase chloroform/methanol 9:1), resolution of the two isomers taking place at the same time. 2-0-(3-Methoxycarbonyl-1,2,4-triazol-1-yl-methyl)-5-0-(4-toluoyl)-isosorbide is obtained by evaporation of the corresponding fractions. Yield: 9.9 g; m.p. 138-139 °C; $[\alpha]_D^{20}$ + 53 (c = 2, methylene chloride). In addition, 7.8 g of 2-0-(5-methoxycarbonyl-1,2,4-triazol-1-yl-methyl)-5-0-(4-toluyl)-isosorbide are isolated; m.p. 110 °C; $[\alpha]_D^{20}$ + 48 (c = 2, methylene chloride).
$C_{19}H_{21}N_3O_7$ MW 403.40

b. 2-0-(3-Methoxycarbonyl-1,2,4-triazol-1-yl-methyl)-isosorbide

6 ml of 30% strength sodium methylate solution are added to 8 g of 2-0-(3-methoxycarbonyl-1,2,4-triazol-1-yl-methyl)-5-0-(4-toluoyl)-isosorbide, dissolved in 110 ml of methanol, and the mixture is stirred at room temperature until complete reaction is recognizable in the thin layer chromatogram (1 hour). The mixture is neutralized by stirring with 60 ml of Amberlite IR-120 ($H^+$, methanol-washed), the exchanger is filtered off with suction, the filtrate is concentrated in vacuo and the oily residue is recrystallized from methanol/ether. Yield: 2.6 g; m.p. 109-110 °C; $[\alpha]_D^{20}$ + 40.5 (c - 1, water).
$C_{11}H_{15}N_3O_6$ MW 285.26

Example 19

2-0-(5-Methoxycarbonyl-1,2,4-triazol-1-yl-methyl)-isosorbide

8 g of 2-0-(5-methoxycarbonyl-1,2,4-triazol-1-yl-methyl)-5-0-(4-toluoyl)-isosorbide (preparation in Example 18 a.) are dissolved in 110 ml of methanol and the solution is stirred with 3 ml of 30% strength sodium methylate solution until, according to the thin layer chromatogram, the transesterification has ended. The mixture is neutralized by addition of 30 ml of Amberlite IR-120 ($H^+$, methanol-washed), the exchanger is filtered off, the filtrate is concentrated in vacuo to a large degree and the resulting oil is stirred with 100 ml of diethyl ether, crystallization occurring. The crude product is recrystallized from methanol/diethyl ether. Yield: 3 g; m.p. 106 ° C; $[\alpha]_D^{20}$ + 37.5 (c = 1, water).
$C_{11}H_{15}N_3O_6$ MW 285.26

Example 20

2-0-(3-Carbamoyl-1,2,4-triazol-1-yl-methyl)-isosorbide

Method 1

8 g of 2-0-(3-methoxycarbonyl-1,2,4-triazol-1-yl-methyl)-5-O-(4-toluoyl)-isosorbide (preparation in Example 18 a.) are suspended in 60 ml of methanol, and 7.5 g of ammonia are passed in at 20-25 ° C, with cooling, solution occurring. After about 22 hours, a precipitate starts to separate out. After 27-30 hours, the precipitate is filtered off with suction and rinsed with methanol and diethyl ether. The crude product is recrystallized from 90% strength aqueous methanol. Yield: 3.7 g; m.p. 173 ° C; $[\alpha]_D^{20}$ + 38 (c = 1, water).
$C_{10}H_{14}N_4O_5$ MW 270.25

Method 2

a. 2-0-Acetoxymethyl-5-0-(4-toluoyl)-isosorbide

39 g of 2-0-chloromethyl-5-0-(4-toluoyl)-isosorbide (Example 1 a.) are dissolved in 400 ml of N,N-dimethylformamide, and 19 g of anhydrous sodium acetate are added. The mixture is stirred at room temperature for 2 days and concentrated in vacuo as far as possible, the residue is dissolved in 500 ml of methylene chloride, undissolved portions are filtered off with suction and the filtrate is extracted twice with 500 ml of water each time. The organic phase is separated off and dried over magnesium sulfate. After filtration and evaporation in vacuo, a viscous oil is obtained, which crystallizes after some time. The compound is reacted further as the crude substance. Yield: 36.7 g. An analytically pure product can be obtained by recrystallization from methanol/water 2:1. m.p. 61-64 ° C; $[\alpha]_D^{20}$ + 37 (c = 2), methylene chloride).
$C_{17}H_{20}O_7$ MW 336.35

b. 2-0-(3-Methoxycarbonyl-1,2,4-triazol-1-yl-methyl)-5-O-(4-toluyl)-isosorbide

33.3 g of 2-0-acetoxymethyl-5-0-(4-toluoyl)-isosorbide are warmed to 140 ° C and 12.5 g of methyl 1,2,4-triazole-3-carboxylate and then 0.27 g of bis-(4-nitrophenyl) phosphate are added to the resulting melt, with stirring. A vacuum is applied and the acetic acid formed is distilled off. After 1 hour, the mixture is cooled and the vitreous residue is recrystallized from methanol. Yield: 8.5 g. The data of the product are identical to those of the compound described in Example 18 a.

c. 2-0-(3-Carbamoyl-1,2,4-triazol-1-yl-methyl)-isosorbide

6.8 g of 2-0-(3-methoxycarbonyl-1,2,4-triazol-1yl-methyl)-5-0-(4-toluoyl)-isosorbide are reacted with methanolic ammonia as described in method 1. Yield: 2.0 g. The data of the compound are identical to

those of the substance described in that method.

Example 21

2-0-(5-Carbamoyl-1,2,4-triazol-1-yl-methyl)-isosorbide

9.5 g of ammonia are passed into a solution of 10 g of 2-0-(5-methoxycarbonyl-1,2,4-triazol-1-yl-methyl)-5-0-(4-toluyl)-isosorbide (Example 18 a.) in 75 ml of methanol at 20-25 ° C. After the mixture has been left to stand at room temperature for two days, a crystalline product separates out and is filtered off with suction and recrystallized from 90% strength aqueous methanol. Yield: 4.4 g; m.p. 158-159 ° C; $[\alpha]_D^{20}$ + 41 (c = 1, water).
$C_{10}H_{14}N_4O_5$ MW 270.25

Example 22

2-0-(5-Azacytosin-1-yl-methyl)-isosorbide

a. 2-0-(5-Azacytosin-1-yl-methyl)-5-0-(4-toluoyl)-isosorbide

25.6 g of crude 2,4-bis-(trimethylsilyl)-5-azacytosine (prepared analogously to Example 7 a. from 11.2 g of 5-azacytosine) are dissolved in 100 ml of 1,2-dichloroethane. 33 g of crude 2-O-chloromethyl-5-0-(4-toluoyl)-isosorbide (Example 1 a.) and then 8 ml of tin tetrachloride are added. The mixture is stirred at room temperature until, according to the thin layer chromatogram, everything has reacted (3-4 hours), and 300 ml of saturated sodium bicarbonate solution are slowly added dropwise. As soon as the evolution of gas has ended, the organic phase is separated off, washed with water, dried over magnesium sulfate and concentrated in vacuo. The vitreous residue is purified by chromatography on silica gel (mobile phase chloroform/methanol 3:1). Yield: 6.4 g; m.p. 212-213 ° C; $[\alpha]_D^{20}$ + 65 (c = 1, methylene chloride).
$C_{18}H_{20}N_4O_6$ MW 388.39

b. 2-0-(5-Azacytosin-1-yl-methyl)-isosorbide

6.4 g of 2-0-(5-azacytosin-1-yl-methyl)-5-0-(4-toluyl)-isosorbide are suspended in 100 ml of methanol, 6 ml of 30% strength sodium methylate solution are added dropwise and the mixture is stirred at room temperature. After about 20 minutes, solution occurs, and after a further 5 minutes a solid precipitates. At this point in time, according to the thin layer chromatogram, the transesterification is complete. The mixture is neutralized by addition of 2.3 ml of glacial acetic acid, cooled to 0 ° C and filtered with suction and the residue is washed with methanol and dried in vacuo at 40 ° C.
Yield: 2.8 g; m.p. 195 ° C; $[\alpha]_D^{20}$ + 41.8 (c = 2, water).
$C_{10}H_{14}N_4O_5$ MW 270.25

Example 23

2-0-(5-Amino-4-carbamoyl-imidazol-1-yl-methyl)-isosorbide

a. 2-0-(5-Amino-4-carbamoyl-imidazol-1-yl-methyl)-5-0-(4-toluyl)-isosorbide

12.6 g of 5-aminoimidazole-4-carboxamide are dissolved in 750 ml of acetonitrile, and 44 ml of triethylamine and 62 g of crude 2-0-chloromethyl-5-0-(4-toluoyl)-isosorbide (Example 1 a.) are added in succession. The mixture is stirred at room temperature until complete reaction is recognizable in the thin

layer chromatogram (about 3 hours). The triethylammonium chloride is filtered off and the filtrate is evaporated in vacuo as far as possible. The residue is dissolved in 400 ml of methylene chloride and the solution is extracted by shaking with 400 ml of water. The title compound is isolated by concentration of the organic phase and recrystallization of the residue from 20% strength aqueous methanol. Yield: 5.7 g; m.p. 142-143 °C; $[\alpha]_D^{20}$ + 34.5 (c = 1, methylene chloride).
$C_{19}H_{22}N_4O_6$ MW 402.42

b. 2-0-(5-Amino-5-carbamoyl-imidazol-1-yl-methyl)-isosorbide

7.4 g of 2-0-(5-amino-4-carbamoyl-imidazol-1-yl-methyl)-5-0-(4-toluoyl)-isosorbide are dissolved in 200 ml of methanol, and 6 ml of 30% strength sodium methylate solution are added. The mixture is stirred until, according to the thin layer chromatogram, everything has reacted (2 hours), neutralized by stirring with 60 ml of Amberlite IR-120 ($H^+$, methanol-washed) and filtered and the filtrate is concentrated in vacuo. The residue is recrystallized from isopropanol. Yield: 2.1 g; m.p. 130-132 °C; $[\alpha]_D^{20}$ + 36.5 (c = 1, water).
$C_{11}H_{16}N_4O_5$ MW 284.28

Example 24

5-0-(5-Iodouracil-1-yl-methyl)-isosorbide

Method 1:

a. 2-0-(4-Toluoyl)-isosorbide

188 g of 5-0-acetyl-isosorbide [Carbohydr. Res. 2, 122 (1966)] are dissolved in a mixture of 500 ml of methylene chloride and 160 ml of pyridine. 170 g of 4-toluyl chloride are added dropwise in the course of about 30 minutes at an internal temperature of 20 °C, while cooling with ice. The mixture is stirred at the same temperature until a thin layer chromatogram indicates complete reaction (1-2 hours), the reaction mixture is poured into 2 l of ice-water and subsequently stirred for 30 minutes and the organic phase is separated off and washed in succession with saturated sodium bicarbonate solution, cold dilute sulfuric acid, sodium bicarbonate solution again and finally water. It is dried over magnesium sulfate and filtered. Concentration in vacuo gives 305 g of 5-0-acetyl-2-0-(4-toluoyl)-isosorbide as a non-crystalline waxy product which is further processed directly:

276 g of the crude product are dissolved in 2.3 l of ethanol, 5 ml of 30% strength sodium methylate solution are added and the mixture is cooled to 10 °C, with stirring. The mixture is stirred at this temperature until, according to the thin layer chromatogram, everything has reacted (2 hours) and is neutralized by addition of 2 ml of glacial acetic acid and the volatile portions are evaporated off in vacuo. The syrup which remains is recrystallized from toluene/petroleum ether. Yield: 139.5 g; m.p. 74 °C; $[\alpha]_D^{20}$ + 65 (c = 2, methylene chloride).
$C_{14}H_{16}O_5$ MW 264.28

b. 5-0-Chloromethyl-2-0-(4-toluoyl)-isosorbide

119 g of 2-0-(4-toluoyl)-isosorbide and 27 g of paraformaldehyde are suspended in 220 ml of methylene chloride and the suspension is cooled to 0 ° and saturated with hydrogen chloride (duration about 2 hours). The solution thus obtained is left to stand at 0 to 2 °C for 15-20 hours, the water of reaction is separated off, the methylene chloride solution is dried over calcium chloride and magnesium sulfate and filtered and the filtrate is concentrated in vacuo. The oil which remains crystallizes on trituration. The crude product is used without further purification. Yield: 144 g. The substance can be obtained in the pure form by recrystallization from methylene chloride/n-hexane. m.p. 100-102 °C; $[\alpha]_D^{20}$ + 157 (c = 2, methylene chloride).
$C_{15}H_{17}ClO_5$ MW 312.76

c. 5-0-(5-Iodouracil-1-yl-methyl)-isosorbide

19.1 g of 2,4-bis-(trimethylsilyl)-5-iodouracil (synthesized analogously to Example 1 b. from 5-iodouracil) and 16.5 g of crude 5-0-chloromethyl-2-0-(4-toluoyl)-isosorbide are dissolved in 50 ml of dry chloroform and the solution is allowed to react at room temperature until a thin layer chromatogram indicates complete reaction (18 hours). The mixture is concentrated in vacuo, the residue is dissolved in 100 ml of methylene chloride and the solution is extracted by stirring with 100 ml of saturated sodium bicarbonate solution for 1 hour, separated off and dried over magnesium sulfate. Filtration and evaporation of the solvent gives 25.3 g of the crude product 5-0-(iodouracil-1-yl-methyl)-2-0-(4-toluoyl)-isosorbide, which is further processed directly:

The crude product is dissolved in 300 ml of methanol, 15 ml of 30% strength sodium methylate solution are added and the mixture is left to stand at room temperature until complete reaction can be seen in the thin layer chromatogram (2 hours). After neutralization with 150 ml of ion exchanger (methanol-washed Amberlite IR-120, $H^+$ form) and filtration, the filtrate is evaporated in vacuo to give a syrup, which slowly crystallizes completely. The pure title compound is obtained by recrystallization from water. Yield: 6.5 g; m.p. 169-170 ° C; $[\alpha]_D^{20}$ + 52 (c = 1, water).
$C_{11}H_{13}IN_2O_6$ MW 396.15

Method 2:

a. 2-0-Acetyl-5-0-chloromethyl-isosorbide

A suspension of 47 g of 2-0-acetyl-isosorbide and 15 g of paraformaldehyde in 120 ml of methylene chloride is saturated with hydrogen chloride at 0 ° C and the resulting solution is left to stand at 0 ° C for 15 to 20 hours. The water which has separated out is removed and the organic phase is dried over calcium chloride and concentrated in vacuo as far as possible. A non-crystallizing oil is obtained. Yield: 59.6 g. The crude compound is reacted further in this form.
$C_9H_{13}ClO_5$ MW 236.66

b. 5-0-(5-Iodouracil-1-yl-methyl)-isosorbide

19.1 g of 2,4-bis-(trimethylsilyl)-5-iodouracil (synthesized analogously to Example 1 b. from 5-iodouracil) and 12.4 g of crude 2-0-acetyl-5-0-chloromethylisosorbide are dissolved in 50 ml of chloroform and the solution is stirred at room temperature until, according to the thin layer chromatogram, the reaction has ended. Thereafter, the mixture is concentrated in vacuo and the residue is taken up in 50 ml of methylene chloride. 50 ml of methanol and then 20 ml of the ion exchanger Dowex 1x2 ($OH^-$, methanol-washed) are added. The mixture is stirred for 15 minutes, the exchanger is filtered off and the filtrate is concentrated in vacuo. The amorphous crude 2-0-acetyl-5-0-(5-iodouracil-1-yl-methyl)-isosorbide (19.5 g) is dissolved in 200 ml of methanol saturated with ammonia at 0 ° C and the solution is left to stand at 0 ° C for 24 hours. A thin layer chromatogram now indicates complete reaction. All the volatile portions are evaporated off in vacuo, the residue is dissolved again in methanol and the solution is evaporated again as far as possible. The vitreous crude product is purified by chromatography on silica gel (mobile phase ethyl acetate), the appropriate fractions are concentrated and the residue is recrystallized from water. Yield: 6.6 g; the data of the product are identical to those of the compound prepared according to method 1.

Example 25

5-0-(3-Carbamoyl-1,2,4-triazol-1-yl-methyl)-isosorbide

a. 5-0-(3-Methoxycarbonyl-1,2,4-triazol-1-yl-methyl)-2-0-(4-toluyl)-isosorbide and 5-O-(5-methoxycarbonyl-1,2,3-triazol-1-yl-methyl)-2-0-(4-toluoyl)-isosorbide

20 g of the crude trimethylsilyl derivative of methyl 1,2,4-triazole-3-carboxylate (prepared analogously to Example 7 a. from 12.7 g of methyl 1,2,4-triazole-3-carboxylate), together with 34 g of crude 5-0-chloromethyl-2-0-(4-toluoyl)-isosorbide (preparation in Example 24, method 1 b.), are dissolved in 100 ml of anhydrous chloroform and the solution is left to stand at room temperature for 18 to 20 hours, until a thin layer chromatogram indicates complete reaction. The mixture is concentrated in vacuo, the syrupy residue is dissolved in 200 ml of methylene chloride, 20 ml of methanol and 200 ml of saturated sodium bicarbonate solution are added and the mixture is stirred until the evolution of gas has ended. The organic layer is separated off, dried over magnesium sulfate and evaporated in vacuo. The crude product is purified by column chromatography on silica gel; the isomers are resolved at the same time. 5-0-(3-Methoxycarbonyl-1,2,4-triazol-1-yl-methyl)-2-0-(4-toluoyl)-isosorbide is isolated by concentrating the appropriate fractions. Yield: 16.1 g; m.p. 88-89 ° C; $[\alpha]_D^{20}$ + 113 (c = 2, methylene chloride).

8.8 g of oily 5-0-(5-methoxycarbonyl-1,2,4-triazol-1-yl-methyl)-2-0-(4-toluyl)-isosorbide are thereby obtained.

$C_{19}H_{21}N_3O_7$ MW 403.40

b. 5-0-(3-Carbamoyl-1,2,4-triazol-1-yl-methyl)-isosorbide

12.1 g of 5-0-(3-methoxycarbonyl-1,2,4-triazol-1-yl-methyl)-2-0-(4-toluyl)-isosorbide are dissolved in 90 ml of methanol, and 11.5 g of ammonia are passed in at a maximum temperature of 25 ° C. The mixture is stirred at room temperature for 20 hours and cooled to 0 to 5 ° C and the precipitate formed is filtered off with suction. 7.7 g of crude 5-0-(3-carbamoyl-1,2,4-triazol-1-yl- methyl)-2-0-(4-toluyl)-isosorbide are isolated and are further reacted directly:

The compound is suspended in 200 ml of methanol, 10 ml of 30% strength sodium methylate solution are added and the mixture is stirred at room temperature until transesterification is complete (1 hour, according to the thin layer chromatogram). After neutralization with 100 ml of Amberlite IR-120 ($H^+$, methanol-washed), the solvent is evaporated off in vacuo, the solid residue is stirred with diethyl ether and filtered off with suction and the product is dried in vacuo. Yield: 4.1 g; m.p. 145-147 ° C; $[\alpha]_D^{20}$ + 80 (c = 1, water).

$C_{10}H_{14}N_4O_5$ MW 270.25

Example 26

5-0-(5-Carbamoyl-1,2,4-triazol-1-yl-methyl)-isosorbide

8.1 g of 5-0-(5-methoxycarbomyl-1,2,4-triazol-1-yl-methyl)-2-0-(4-toluoyl)-isosorbide (preparation in Example 25 a.) are dissolved in 60 ml of methanol, and 7.7 g of ammonia are passed in at 20 to 25 ° C. After the mixture has been left to stand at room temperature for 4 days, the volatile portions are evaporated off in vacuo and the residue is recrystallized from methanol.

Yield: 3.5 g; m.p. 132-133 ° C; $[\alpha]_D^{20}$ + 72 (c = 1, water).

$C_{10}H_{14}N_4O_5$ MW 270.25

Example 27

5-0-(5-Azacytosin-1-yl-methyl)-isosorbide

a. 5-0-(5-Azacytosin-1-yl-methyl)-2-0-(4-toluyl)-isosorbide

25.6 g of crude 2,4-bis-(trimethylsilyl)-5-azacytosine (prepared analogously to Example 7 a. from 11.2 g of 5-azacytosine) are dissolved in 300 ml of 1,2-dichloroethane. 8 ml of tin tetrachloride and 33 g of crude 5-0-chloromethyl-2-0-(4-toluoyl)-isosorbide (synthesis in Example 24, Method 1 b.) are added. The mixture is stirred until everything has dissolved and is left to stand until, according to the thin layer chromatogram, a complete reaction has taken place (18 hours). 300 ml of saturated sodium bicarbonate solution are now

added dropwise, with vigorous stirring, and the mixture is stirred until the evolution of gas has ended. The organic phase is separated off and concentrated in vacuo as far as possible and the crude product which remains is purified by chromatography on silica gel (mobile phase chloroform/methanol 9:1). The appropriate fractions are concentrated in vacuo and the residue is recrystallized from isopropanol. Yield: 5.2 g; m.p. 215-218 °C; $[\alpha]_D^{20}$ + 56 (c = 0.5, methylene chloride).
$C_{18}H_{20}N_4O_6$ MW 388.39

b. 5-0-(5-Azacytosin-1-yl-methyl)-isosorbide

5.2 g of 5-0-(5-azacytosin-1-yl-methyl)-2-0-(4-toluoyl)-isosorbide are dissolved in 100 ml of methanol, and 5 ml of 30% strength sodium methylate solution are added. The mixture is stirred at room temperature until a thin layer chromatogram indicates complete transesterification, and is neutralized by addition of 50 ml of ion exchanger Amberlite IR-120 ($H^+$, methanol-washed) and filtered and the filtrate is evaporated to dryness in vacuo. The residue is recrystallized from ethanol. Yield: 1.8 g; m.p. 160-162 °C; $[\alpha]_D^{20}$ + 60 (c = 1, water).
$C_{10}H_{14}N_4O_5$ MW 270.25

Example 28

5-0-(Uracil-1-yl-methyl)-isosorbide

a. 2-0-Benzyl-5-0-chloromethyl-isosorbide

A suspension of 56.9 g of 2-0-benzyl-isosorbide (U.S. Patent 4,169,152) and 15.6 g of paraformaldehyde in 120 ml of methylene chloride is cooled to 0 °C and is saturated with hydrogen chloride at this temperature. The resulting solution is left to stand at 0 to 2 °C for 15 to 20 hours, the water which has separated out is removed, the organic phase is dried over calcium chloride and magnesium sulfate and filtered and the filtrate is concentrated in vacuo. The viscous oil which remains is further reacted as the crude product. Yield: 70 g.
$C_{14}H_{17}ClO_4$ MW 284.74

b. 2-0-Benzyl-5-0-(uracil-1-yl-methyl)-isosorbide

25.8 g of 2,4-bis-(trimethylsilyl)-uracil (prepared from uracil analogously to Example 1 b.) are dissolved in 100 ml of dry chloroform, 28.5 g of crude 2-0-benzyl-5-0-chloromethyl-isosorbide are added and the solution is stirred at room temperature until reaction is complete (3 hours, according to the thin layer chromatogram). All the volatile portions are evaporated off in vacuo, the residue is dissolved in 100 ml of methylene chloride, the solution is stirred with 100 ml of saturated sodium bicarbonate solution until the evolution of gas has ended, the lower layer is separated off and concentrated in vacuo and the oil which remains is purified by chromatography on silica gel (mobile phase chloroform/methanol (9:1). The pure compound is obtained as a non-crystallizing resin.
Yield: 25.6 g; $[\alpha]_D^{20}$ + 58 (c = 2, methylene chloride).
$C_{18}H_{20}N_2O_6$ MW 360.38

c. 5-0-(Uracil-1-yl-methyl)-isosorbide

11 g of 2-0-benzyl-5-0-(uracil-1-yl-methyl)-isosorbide are dissolved in 150 ml of methanol, 2 g of palladium-on-charcoal (5%) are added and the mixture is hydrogenated at room temperature under normal pressure. As soon as the uptake of hydrogen has ended, the catalyst is filtered off and the filtrate is concentrated to dryness in vacuo. The residue is recrystallized from methanol. Yield: 3.3 g; m.p. 136-137 °C; $[\alpha]_D^{20}$ + 65 (c = 2, water).
$C_{11}H_{14}N_2O_6$ MW 270.25

Example 29

2-0-(3-Carbamoyl-1,2,4-triazol-1-yl-methyl)-isomannide

a. 2-0-Benzoyl-5-0-chloromethyl-isomannide

175.2 g of 2-0-benzoyl-isomannide (German Patent 3,123,719) and 42.5 g of paraformaldehyde are suspended in 350 ml of methylene chloride, and hydrogen chloride is passed in at 0 ° C until the suspension is saturated. After the mixture has been left to stand at 0 to 2 ° C for 18 to 20 hours, the water of reaction which has separated out is removed, the mixture is dried over calcium chloride and then magnesium sulfate and filtered and the filtrate is concentrated in vacuo as far as possible. viscous oil which does not crystallize is obtained. The crude compound is further processed in this form. Yield: 218 g.
$C_{14}H_{15}ClO_5$ MW298.73

b. 2-0-Benzoyl-5-0-(3-methoxycarbonyl-1,2,4-triazol-1-yl-methyl)-isomannide and 2-0-benzoyl-5-0-(5-methoxycarbonyl-1,2,4-triazol-1-yl-methyl)-isomannide

20 g of the crude trimethylsilyl compound of methyl 1,2,4-triazole-3-carboxylate (prepared analogously to Example 7 a. from 12.7 g of methyl 1,2,4-triazole-3-carboxylate) and 31 g of crude 2-0-benzoyl-5-0-chloromethyl-isomannide are dissolved in 100 ml of dry chloroform and the solution is left to stand at room temperature until complete reaction can be seen in the thin layer chromatogram (17 hours). A slight cloudiness is filtered off, the filtrate is evaporated in vacuo, the syrup is dissolved in 100 ml of methylene chloride and the solution is extracted by stirring with 50 ml of sodium bicarbonate solution for 2 hours and then with 100 ml of water and evaporated in vacuo. The residue is purified by chromatography on silica gel (mobile phase chloroform/methanol 95:5); the two isomers are separated at the same time. 2-0-Benzoyl-5-0-(3-methoxycarbonyl-1,2,4-triazol-1-yl-methyl)-isomannide is obtained as a solid after recrystallization from ethanol. Yield: 8.1 g; m.p. 125-126 ° C; $[\alpha]_D^{20}$ + 113.8 (c = 2, methylene chloride).

In addition, 2-0-benzoyl-5-0-(5-methoxycarbonyl-1,2,4-triazol-1-yl-methyl)-isomannide is isolated as a viscous oil. Yield: 12.4 g; $[\alpha]_D^{20}$ + 98.5 (c = 2, methylene chloride).
$C_{18}H_{19}N_3O_7$MW 389.38

c. 2-0-(3-Carbamoyl-1,2,4-triazol-1-yl-methyl)-isomannide

7.5 g of 2-0-benzoyl-5-0-(3-methoxycarbonyl-1,2,4-triazol-1-yl-methyl)-isomannide are suspended in 70 ml of methanol, and 7.2 g of ammonia are passed in at a maximum temperature of 25 ° C, solution gradually taking place. The mixture is left to stand at room temperature until the reaction is complete (16 hours, according to the thin layer chromatogram) and is concentrated to dryness in vacuo. The solid residue is recrystallized from ethanol. Yield: 4.1 g; m.p. 135.5-137.5 ° C; $[\alpha]_D^{20}$ + 104 (c = 2, water).
$C_{10}H_{14}N_4O_5$ MW 270.25

Example 30

2-0-(5-Carbamoyl-1,2,4-triazol-1-yl-methyl)-isomannide

11 g of 2-0-benzoyl-5-0-(5-methoxycarbonyl-1,2,4-triazol-1-yl-methyl)-isomannide (synthesis in Example 29 b.) are dissolved in 80 ml of methanol, and 10.5 g of ammonia are passed in at a maximum temperature of 25 ° . The mixture is left to stand at 20-25 ° C for 18-20 hours. A thin layer chromatogram now indicates that the reaction is complete. The mixture is concentrated in vacuo and the solid is recrystallized from methanol. Yield: 3.8 g; m.p. 144-145 ° C; $[\alpha]_D^{20}$ + 93.5 (c = 2, water).
$C_{10}H_{14}N_4O_5$ MW 270.25

Example 31

2-0-(5-Iodouracil-1-yl-methyl)-isomannide

a. 2-0-Benzoyl-5-0-(5-iodouracil-1-yl-methyl)-isomannide

19.1 g of 2,4-bis-(trimethylsilyl)-5-iodouracil (prepared analogously to Example 1 b. from 5-iodouracil) and 31 g of crude 2-0-benzoyl-5-0-chloromethyl-isomannide (Example 29 a.) are dissolved in 100 ml of dry chloroform and the solution is left to stand at room temperature until everything has reacted (after 5 days, in the thin layer chromatogram). The volatile constituents are evaporated off in vacuo, the syrup which remains is dissolved in 100 ml of methylene chloride and the solution is stirred with 50 ml of sodium bicarbonate solution for 2 hours. After the organic layer has been separated off and concentrated, a viscous oil is obtained, and is purified by chromatography on silica gel (mobile phase chloroform/methanol 9:1). The appropriate fractions are evaporated in vacuo and recrystallized from toluene. Yield: 10.7 g; m.p. 150-152 °C; $[\alpha]_D^{20}$ + 110.8 (c = 2, methylene chloride).
$C_{18}H_{17}IN_2O_7$ MW 500.26

b. 2-0-(5-Iodouracil-1-yl-methyl)-isomannide

9.5 g of 2-0-benzoyl-5-0-(5-iodouracil-1-yl- methyl)-isomannide, suspended in 100 ml of methanol, are stirred with 5 ml of 30% strength sodium methylate solution until solution and complete transesterification have occurred (1 hour, according to the thin layer chromatogram). The mixture is neutralized by addition of 50 ml of methanol-washed ion exchanger (Amberlite IR-120, $H^+$) and filtered, the filtrate is evaporated in vacuo and the vitreous residue is dissolved in hot isopropanol. On cooling, an amorphous precipitate separates out and is filtered off, the solid is dissolved in water and the solution is freeze-dried. Yield: 5 g; m.p. 60-80 °C; $[\alpha]_D^{20}$ + 62.5 (c = 2, water).
$C_{11}H_{13}IN_2O_6$ MW 396.15

Example 32

2-0-(5-Iodouracil-1-yl-methyl)-isoidide

a. 2-0-Benzoyl-5-0-chloromethyl-isoidide

62.5 g of 2-0-benzoyl-isoidide [Carboh. Res. 64, 135 (1978)] and 15.2 g of paraformaldehyde, suspended in 150 ml of methylene chloride, are saturated with hydrogen chloride at 0 °C. The mixture is left to stand at 0 to 2 °C for 18-20 hours, the water formed is separated off, the mixture is dried over calcium chloride and magnesium sulfate and filtered and the filtrate is concentrated in vacuo as far as possible. An oil, which is further reacted in this form, is obtained. Yield: 70.6 g.
$C_{14}H_{15}ClO_5$ MW 298.73

b. 2-0-8enzoyl-5-0-(5-iodouracil-1-yl-methyl)-isoidide

A solution of 38.2 g of 2,4-bis-(trimethylsilyl)-5-iodouracil (prepared analogously to Example 1 b. from 5-iodouracil) and 30 g of crude 2-0-benzoyl-5-0-chloromethyl-isoidide in 100 ml of anhydrous chloroform is stirred at room temperature until a thin layer chromatogram indicates complete reaction (20 hours). All the volatile portions are evaporated off in vacuo, the residue is dissolved in 100 ml of methylene chloride and the solution is extracted by stirring with 50 ml of saturated sodium bicarbonate solution. The organic layer is dried over magnesium sulfate and filtered, the filtrate is concentrated in vacuo and the residue is recrystallized from isopropanol. Yield: 39.1 g; m.p. 80-85 °C; $[\alpha]_D^{20}$ + 33.8 (c = 2, methylene chloride).
$C_{18}H_{17}IN_2O_7$MW 500.26

c. 2-0-(5-Iodouracil-1-yl-methyl)-isoidide

36.7 g of 2-0-benzoyl-5-0-(5-iodouracil-1-yl-methyl)-isoidide are dissolved in 300 ml of methanol, and 20 ml of sodium methylate are added. The mixture is stirred until, according to the thin layer chromatogram, everything has reacted (15 minutes) and is neutralized with 200 ml of Amberlite IR-120 ($H^+$, methanol-washed), the exchanger is filtered off, the filtrate is concentrated in vacuo and the residue is recrystallized from 90% strength aqueous methanol. Yield: 17.5 g; m.p. 115-117° C; $[\alpha]_D^{20}$ + 22.5 (c = 1, methanol).
$C_{11}H_{13}IN_2O_6$ . $H_2O$MW 414.17

Example 33

2-0-(Carbamoyl-1,2,4-triazol-1-yl-methyl)-isoidide

a. 2-0-Benzoyl-5-0-(3-methoxycarbonyl-1,2,4-triazol-1-yl-methyl)-isoidide and 2-0-benzoyl-5-0-(5-methoxycarbonyl-1,2,4-triazol-1-yl-methyl)-isoidide

20 g of the crude trimethylsilyl compound of methyl 1,2,4-triazole-3-carboxylate (synthesized from 12.7 g of methyl 1,2,4-triazole-3-carboxylate analogously to Example 7 a.) and 30 g of crude 2-0-benzoyl-5-0-chloromethyl-isoidide (Example 32 a.) are stirred in 100 ml of anhydrous chloroform until complete reaction is detectable in the thin layer chromatogram (20 hours). The mixture is concentrated in vacuo, the residue is dissolved in 100 ml of methylene chloride, the solution is extracted by stirring with 50 ml of saturated sodium bicarbonate solution for 2 hours and the methylene chloride layer is separated off and evaporated in vacuo as far as possible. The viscous oil which remains is purified by chromatography on silica gel (mobile phase chloroform/methanol 9:1) and the two isomers are resolved at the same time. 2-0-Benzoyl-5-0-(5-methoxycarbonyl-1,2,4-triazol-1-yl-methyl)-isoidide is obtained as a crystalline product. Yield: 7.2 g; m.p. 90-90.5° C; $[\alpha]_D^{20}$ + 44.8 (c = 2, methylene chloride).

2-0-Benzoyl-5-0-(3-methoxycarbonyl-1,2,4-triazol-1-yl-methyl)-isoidide is also obtained, as an oil. Yield: 14.3 g; $[\alpha]_D^{20}$ + 17.3 (c = 2, methylene chloride).
$C_{18}H_{19}N_3O_7$ MW 389.38

b. 2-0-(3-Carbamoyl-1,2,4-triazol-1-yl-methyl)-isoidide

A solution of 14 g of 2-0-benzoyl-5-0-(3-methoxycarbonyl-1,2,4-triazol-1-yl-methyl)-isoidide in 100 ml of methanol is saturated with ammonia at a maximum temperature of 25° C and the mixture is left to stand at room temperature until a thin layer chromatogram indicates complete reaction (2 days). The volatile constituents are distilled off in vacuo until a volume of about 50 ml is reached and the solid which has precipitated is filtered off with suction and recrystallized from methanol. Yield: 5.3 g; m.p. 192.5-193° C; $[\alpha]_D^{20}$ + 7 (c = 1, water).
$C_{10}H_{14}N_4O_5$ MW 270.25

Example 34

2-0-(5-Carbamoyl-1,2,4-triazol-1-yl-methyl)-isoidide

7 g of 2-0-benzoyl-5-0-(5-methoxycarbonyl-1,2,4-triazol-1-yl-methyl)-isoidide are suspended in 50 ml of methanol, and ammonia is passed in at a maximum temperature of 25° C until the suspension is saturated. After 2 days, everything has reacted, according to the thin layer chromatogram. The mixture is concentrated and the residue is purified by chromatography on silica gel (mobile phase chloroform/methanol 4:1). The appropriate fractions are concentrated and the crude product is recrystallized from ethanol. Yield: 3.1 g; m.p. 126-126.5° C; $[\alpha]_D^{20}$ + 16.5 (c = 2, water).
$C_{10}H_{14}N_4O_5$ MW 270.25

Example 35

2-0-(5-Methyluracil-1-yl-methyl)-isosorbide-5-monophosphoric acid

11 g of 2-0-(5-methyluracil-1-yl-methyl)-isosorbide (Example 5) are suspended in 40 ml of acetonitrile, 11 ml of phosphorus oxytrichloride are added and the mixture is cooled to 5-10 °C. A solution of 3 ml of pyridine in 10 ml of acetonitrile is now added at a maximum temperature of 10 °C and the mixture is allowed to warm slowly to room temperature. Solution occurs at 17-18 °C. As soon as a thin layer chromatogram indicates complete reaction (30 minutes), the reaction mixture is poured onto 1 l of ice-water, 110 g of active charcoal (Carboraffin P) are added and the mixture is stirred until, according to the thin layer chromatogram, all the UV-absorbing constituents are bonded to the charcoal. The active charcoal is filtered off with suction and washed with water until free from chloride and phosphate. Elution is now carried out with a total of 2-3 l cf 3% strength aqueous-methanolic ammonia (water/methanol 9:1), until a sample of the eluate no longer shows UV absorption. The combined eluates are concentrated in vacuo as far as possible, the syrup which remains is dissolved in 90% strength aqueous methanol and the product is precipitated with ethanol. The pure title compound is obtained as a crystalline diammonium salt. Yield: 7 g; m.p. decomp. > 110 °; $[\alpha]_D^{20}$ + 29 (c = 1, water).
$C_{12}H_{23}N_4O_9P$ MW 398.32

Example 36

2-0-(4-Hydroxy-1H-pyridin-2-on-1-yl-methyl)-isosorbide

a. 2-0-(4-Hydroxy-1H-pyridin-2-on-1-yl-methyl)-5-0-(4-toluoyl)-isosorbide

12.8 g of 2,4-bis-(trimethylsilyloxy)-pyridine (prepared from 4-hydroxy-2-pyridone and hexamethyldisilazane analogously to Example 1 b.) and 16.5 g of crude 2-0-chloromethyl-5-0-(4-toluoyl)-isosorbide (Example 1 a.) are dissolved in 100 ml of dry chloroform and the solution is stirred at room temperature until a thin layer chromatogram indicates complete reaction (4 hours). The mixture is concentrated in vacuo, the residue is dissolved in methanol, the solution is concentrated again, the crude product is purified by chromatography on silica gel (mobile phase chloroform/methanol 9:1) and the product is recrystallized from ethyl acetate. Yield: 10.5 g; m.p. 100-101 °C (decomp.); $[\alpha]_D^{20}$ + 48.5 (c = 1, methanol).
$C_{20}H_{21}NO_7$ MW 387.40

b. 2-0-(4-Hydroxy-1H-pyridin-2-on-1-yl-methyl)-isosorbide

6.7 g of 2-0-(4-hydroxy-1H-pyridin-2-on-1-yl-methyl)-5-0-(4-toluoyl)-isosorbide and 4 ml of 30% strength sodium methylate solution are dissolved in 50 ml of methanol and the solution is stirred until transesterification is complete (1 hour, according to the thin layer chromatogram). The mixture is neutralized by addition of 40 ml of methanol-washed Amberlite IR-120 ($H^+$), the exchanger is filtered off with suction and the filtrate is concentrated to dryness in vacuo. The residue is recrystallized from ethanol. Yield: 2.8 g; m.p. 178-179 °C; $[\alpha]_D^{20}$ + 43.5 (c = 1, water).
$C_{12}H_{15}NO_6$ MW 269.26

Example 37

5-0-(5-Methyluracil-1-yl-methyl)-isosorbide

a. 5-0-(5-Methyluracil-1-yl-methyl)-2-0-(4-toluoyl)-isosorbide

13.6 g of 2,4-bis-(trimethylsilyl)-5-methyluracil (preparation from 5-methyluracil analogously to Example 1 b.) are dissolved in 50 ml of dry chloroform, and 16.3 g of crude 5-0-chloromethyl-2-0-(4-toluoyl)-isosorbide (Example 24, method 1 b.) are added. The mixture is stirred until complete reaction can be seen

in the thin layer chromatogram (4 hours), the mixture is concentrated to a syrup in vacuo, the syrup is dissolved in 50 ml of methylene chloride and the solution is stirred with 20 ml of saturated sodium bicarbonate solution until the evolution of gas has ended. The organic phase is separated off and concentrated in vacuo. The residue is dissolved in methanol and the solution is evaporated again as far as possible. 19 g of crude product, which is further processed without purification, are obtained.
$C_{20}H_{22}N_{2}0_{7}$ MW 402.41

b. 5-0-(5-Methyluracil-1-yl-methyl)-isosorbide

19 g of crude 5-0-(5-methyluracil-1-yl-methyl)-2-0-(4-toluoyl)-isosorbide are dissolved in 50 ml of methanol, and 5 ml of 30% strength sodium methylate solution are added. The mixture is stirred until a thin layer chromatogram indicates complete reaction (1.5 hours) and is neutralized by addition of 50 ml of ion exchanger (Amberlite IR-120, $H^{+}$, methanol-washed) and filtered and the filtrate is concentrated in vacuo. The oily residue is purified by chromatography on silica gel (mobile phase chloroform/methanol 9:1). Concentration of the corresponding fractions gives a vitreous product which is dissolved in water and freeze-dried. Yield: 2.6 g; m.p. 48-55 °C; $[\alpha]_{D}^{20}$ + 69.5 (c = 1, water).
$C_{12}H_{16}N_{2}0_{6}$ . 0.5 $H_{2}0$MW 293.28

Example 38

2-0-(5-Methyluracil-1-yl-methyl)-isomannide

11.3 g of 2,4-bis-(trimethylsilyl)-5-methyluracil (prepared analcgously to Example 1 b. from 5-methyluracil) and 13.3 g of crude 2-0-benzoyl-5-0-chloromethyl-isomannide (Example 29 a.) are stirred in 50 ml of chloroform until complete reaction can be seen in the thin layer chromatogram (17 hours). After concentrating the mixture in vacuo and dissolving the residue in 50 ml of methylene chloride, the solution is extracted by stirring with 20 ml of saturated sodium bicarbonate solution for one hour, the organic phase is separated off and concentrated in vacuo, the residue is dissolved in methanol, the solution is concentrated again and the residue of crude 2-0-benzoyl-5-0-(5-methyluracil-1-yl-methyl)-isomannide is taken up in 50 ml of methanol.

8 ml of 30% strength sodium methylate solution are added, the mixture is stirred until a thin layer chromatogram demonstrates complete transesterification, the mixture is neutralized with 80 ml of Amberlite IR-120 ($H^{+}$, methanol-moist) and the exchanger is filtered off with suction. The filtrate is concentrated in vacuo and the vitreous crude product is purified by chromatography on silica gel (mobile phase chloroform/methanol 9:2). Recrystallization from ethyl acetate gives the pure title compound. Yield: 4.5 g; m.p. 141-142.5 °C; $[\alpha]_{D}^{20}$ + 92 (c = 1, water).
$C_{12}H_{16}N_{2}0_{6}$MW 284.27

Example 39

2-0-(5-Methyluracil-1-yl-methyl)-isoidide

8.5 g of 2,4-bis-(trimethylsilyl)-5-methyluracil (preparation analogously to Example 1 b. from 5-methyluracil) and 9.1 g of crude 2-0-benzoyl-5-0-chloromethylisoidide (Example 32 a.), dissolved in 50 ml of dry chloroform, are stirred at room temperature until a thin layer chromatogram indicates complete reaction (15 hours). The mixture is concentrated in vacuo, the residue is dissolved in 50 ml of methylene chloride, the solution is stirred with 20 ml of sodium bicarbonate solution until the evolution of gas has ended, the organic layer is separated off and concentrated in vacuo, the residue is subsequently evaporated with methanol and the crude 2-0-benzoyl-5-0-(5-methyluracil-1-yl-methyl)-isoidide is dissolved in 100 ml of methanol.

After addition of 6 ml of 30% strength sodium methylate solution, the mixture is stirred until complete transesterification can be seen in the thin layer chromatogram (30 minutes). It is neutralized by stirring with 60 ml of Amberlite IR-120 ($H^{+}$, methanol-washed) and filtered and the filtrate is concentrated in vacuo as far

as possible. The residue is recrystallized from ethyl acetate. Yield: 5.3 g; m.p. 163-165 °C; $[\alpha]_D^{20}$ + 16 (c = 1, water)
$C_{12}H_{16}N_2O_6$ MW 284.27

## Claims

1. Isohexide nucleosides of the general formula I

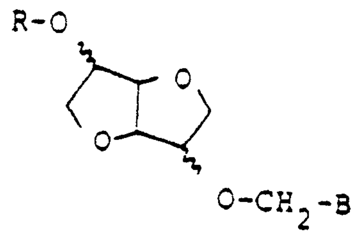

in which the bond between the ring system and the substituents can be either endo- or exocyclic in position, and in which
R
denotes hydrogen; straight-chain or branched aliphatic acyl with 2 to 5 carbon atoms; benzoyl optionally substituted with halogen, alkyl having up to 6 carbon atoms or nitro; benzyl or a phosphate radical;
and B
represents a nitrogen-containing heterocyclic radical, selected from the group comprising
a) uracils of the general formula II

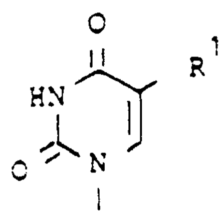

II

wherein $R^1$ denotes hydrogen or halogen or straight-chain or branched $C_1$-$C_6$-alkyl, vinyl, allyl or ethynyl which may be substituted by hydroxyl groups or one or more halogens;
b) cytosines of the general formula III

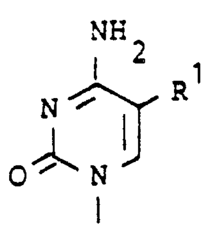

III

in which $R^1$ has the above meaning;
c) isocytosines of the general formula IV

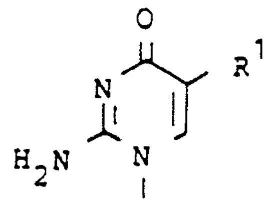

IV

in which $R^1$ has the above meaning;

d) 5-azacytosine of the formla V

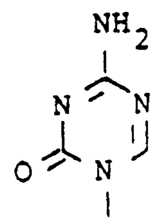

V

e) triazoles of the general formula VI

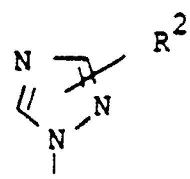

VI

in which $R^2$ occupies either the 3- or the 5-position and may have one of the following meanings: hydrogen; alkoxycarbonyl -COOR$^3$, in which $R^3$ denotes straight-chain or branched $C_1$-$C_5$-alkyl; carboxamide -CO-$NH_2$; thiocarboxamide -CS-$NH_2$ or cyano -CN;

f) imidazoles of the general formula VII

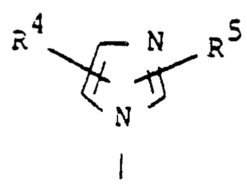

VII

in which the two substituents $R^4$ and $R^5$ are identical or different and denote hydrogen, amino -$NH_2$, carboxamide -CO-$NH_2$, thiocarboxamide -CS-$NH_2$ or cyano -CN, or, if appropriate, a salt thereof with an inorganic or organic acid.

2. Isohexide nucleosides according to claim 1, wherein R denotes acetyl.

3. Isohexide nucleosides according to claim 1, wherin R denotes benzoyl, toluoyl, chlorobenzoyl or nitrobenzoyl.

4. Isohexide nucleosides according to claim 1, in which R represents hydrogen or a phosphate radical.

5. Isohexide nucleosides according to claim 1, in which R represents hydrogen or a phosphate radical and B denotes uracils of the general formula II, cytosines of the general formula III or isocytosines of the general formula IV, wherein $R^1$ in each case represents hydrogen, methyl, ethyl, propyl, isopropyl, butyl, isobutyl, pentyl, hexyl, hydroxymethyl, hydroxyethyl, vinyl, allyl, ethynyl, fluorine, chlorine, bromine, iodine, bromovinyl, iodovinyl or trifluoromethyl; or B denotes triazoles or the general formula VI, wherein $R^2$ represents carboxamide -CO-$NH_2$, methoxycarbonyl -$COOCH_3$ or ethoxycarbonyl -$COOC_2H_5$; or B denotes imidazoles of the general formula VII, wherein one of the substituents $R^4$ and

$R^5$ represents amino $-NH_2$ and the other represents carboxamide $-CO-N_2$, or cyano -CN.

6. Isohexide nucleosides according to one of claims 1 - 5, which are derivatives of the isomannide of the general formula Ia

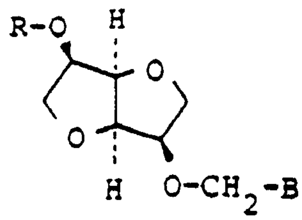

I a

in which R and B have the meaning as defined in claims 1 - 5.

7. Isohexide nucleosides according to one of claims 1 - 5, which are derivatives of the isoidide of the general formula Ib

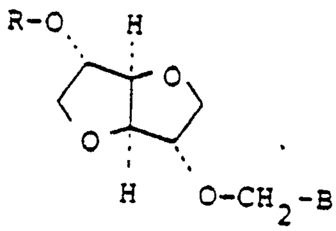

I b

in which R and B have the meaning as defined in claims 1 - 5.

8. Isohexide nucleosides according to one of claims 1 - 5, which are derivatives of the isosorbide of the general formula Ic or Id

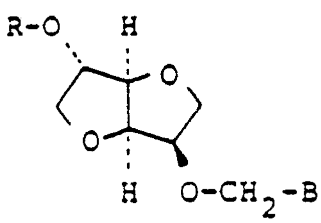

Ic

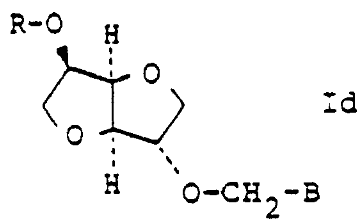

Id

in which R and B have the meaning as defined in claims 1 - 5.

9. An isohexide nucleoside according to claim 1 which is 5-0-(3-Carbamoyl-1, 2, 4-triazol-l-yl-methyl)-isosorbide.

10. An isohexide nucleoside according to claim 1 which is 2-0-(3-Carbamoyl-1, 2, 4-triazol-l-yl-methyl)-isomannide.

11. An isohexide nucleoside according to claim 1 which is 2-0-(5-Carbamoyl-1,2,4-triazol-l-yl-methyl)-isomannide.

12. A process for the preparation of an isohexide nucleoside as claimed in claim 1, which comprises, in a manner which is known per se, in accordance with the following equation

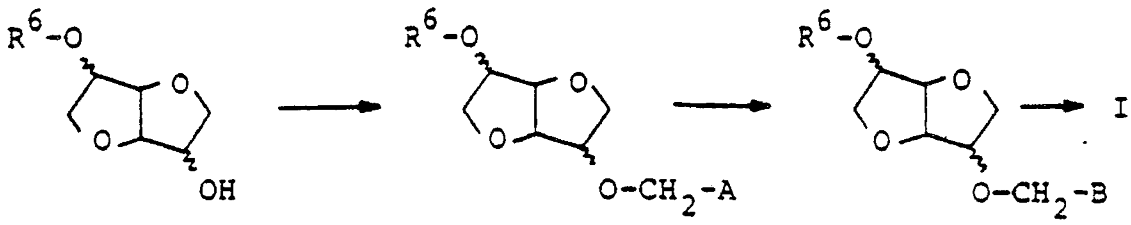


VIII IX X

converting a monosubstituted isohexide of the general formula VIII in which $R^6$ has the same meaning as R, optionally protected by a protective group customary in nucleoside chemistry, into a compound of the general formula IX in which A represents a leaving group or a leaving atom, and converting the compound of the general formula IX into a compound of the general formula X either by reacting it with a nitrogen-containing heterocyclic base of the general formula XI

B - H XI

in which B has the abovementioned meaning, or by reacting it with a protected derivative of this nitrogen-containing heterocyclic base, and if appropriate splitting off the protective group from $R^6$ in the compound of the general formula X and if appropriate converting the compound of the general formula I, in which R denotes hydrogen, into a compound of the general formula I in which R denotes a phosphate radical by reaction with a phosphorylating reagent, preferably with phosphorus oxytrichloride.

**13.** The use of an isohexide nucleoside as claimed in any of claim 1 - 11 for manufacturing a medicament.

**14.** The use of an isohexide nucleoside as claimed in any of claims 1 - 11 for manufacturing a medicament for preventing and combating virus diseases.

**15.** A medicament containing at least one compound as claimed in any of claims 1 - 11 and customary auxiliaries and or excipients.

Claims for the following Contracting States : ES, GR

**1.** Process for the preparation of isohexide nucleosides of the general formula I

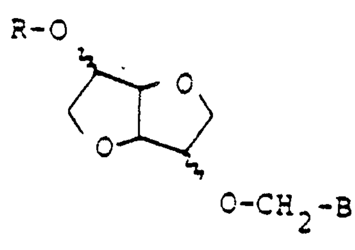

:

in which the bond between the ring system and the substituents can be either endo- or exocyclic in position, and in which

R

denotes hydrogen; straight-chain or branched aliphatic acyl with 2 to 5 carbon atoms; benzoyl optionally subsituted with halogen, alkyl having up to 6 carbon atoms or nitro; benzyl or a phosphate radical;

and B

represents a nitrogen-containing heterocyclic radical, selected from the group comprising

a) uracils of the general formula II

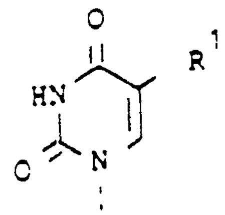

II

wherein $R^1$ denotes hydrogen or halogen or straight-chain or branched $C_1$-$C_6$-alkyl, vinyl, allyl or ethynyl which may be substituted by hydroxyl groups or one or more halogens;

b) cytosines of the general formula III

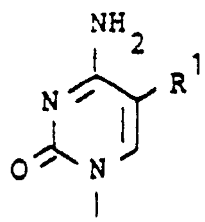

III

in which $R^1$ has the above meaning;

c) isocytosines of the general formula IV

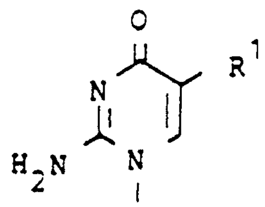

IV

in which $R^1$ has the above meaning;

d) 5-azacytosine of the formla V

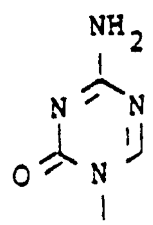

V

e) triazoles of the general formula VI

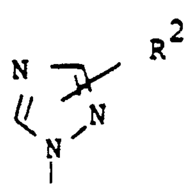

VI

in which $R^2$ occupies either the 3- or the 5-position and may have one of the following meanings: hydrogen; alkoxycarbonyl $-COOR^3$, in which $R^3$ denotes straight-chain or branched $C_1$-$C_5$-alkyl; carboxamide $-CO-NH_2$; thiocarboxamide $-CS-NH_2$ or cyano -CN;

f) imidazoles of the general formula VII

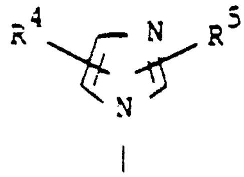

VII

in which the two substituents $R^4$ and $R^5$ are identical or different and denote hydrogen, amino $-NH_2$, carboxamide $-CO-NH_2$, thiocarboxamide $-CS-NH_2$ or cyano -CN, or, if appropriate, a salt thereof with an inorganic or organic acid, which process comprises, in a manner which is known per se, in accordance with the following equation

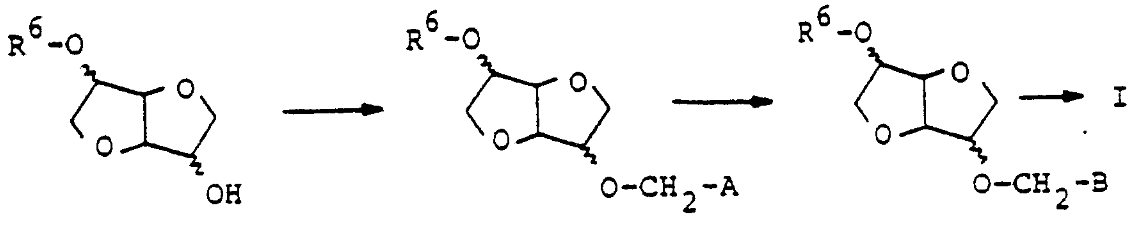

VIII IX X

converting a monosubstituted isohexide of the general formula VIII in which $R^6$ has the same meaning as R, optionally protected by a protective group customary in nucleoside chemistry, into a compound of the general formula IX in which A represents a leaving group or a leaving atom, and converting the compound of the general formula IX into a compound of the general formula X either by reacting it with a nitrogen-containing heterocyclic base of the general formula XI

$$B - H \qquad XI$$

in which B has the abovementioned meaning, or by reacting it with a protected derivative of this nitrogen-containing heterocyclic base, and if appropriate splitting off the protective group from $R^6$ in the compound of the general formula X and if appropriate converting the compound of the general formula I, in which R denotes hydrogen, into a compound of the general formula I in which R denotes a phosphate radical by reaction with a phosphorylating reagent, preferably with phosphorus oxytrichloride.

2. Process according to claim 1, in which A represents chlorine, bromine or iodine or a straight-chain or branched aliphatic $C_2$-$C_5$-acyloxy radical.

3. Process according to claim 1 or 2, in which the protective group in the nitrogen-containing heterocyclic base of the general formula XI comprises aliphatic or aromatic acyl radicals, preferably acetyl or benzoyl.

4. Process according to claim 1 or 2, in which the compound of formula IX is reacted with a silylated derivative of the nitrogen-containing heterocyclic base, a silylated derivative being understood as one which is obtained by reaction of the base of the general formula XI with a trialkylsilyl halide, preferably trimethylsilyl chloride, or a hexaalkyldisilazane, preferably hexamethyl-disilazane, or with a mixture of the two, or is reacted with a metal salt of the nitrogen-containing heterocyclic base, preferably a silver or mercury salt, or if appropriate is reacted with an alkoxy derivative of this nitrogen-containing heterocyclic base, "alkoxy" being understood as a lower alkoxy group, preferably a methoxy or ethoxy group.

5. Process according to any one of claims 1 - 4, in which an isohexide nucleoside of the general formula I is prepared in which R denotes acetyl.

6. Process according to any one of claims 1 - 4, in which an isohexide nucleoside of the general formula I is prepared in which R denotes benzoyl, toluoyl, chlorobenzoyl or nitrobenzoyl.

7. Process according to any one of claims 1 - 4, in which an isohexide nucleoside of the general formula I is prepared in which R represents hydrogen or a phosphate radical.

8. Process according to any one of claims 1 - 4, in which an isohexide nucleoside of the general formula I is prepared in which R represents hydrogen or a phosphate radical and B denotes uracils of the general formula II, cytosines of the general formula III or isocytosines of the general formula IV, wherein $R^1$ in each case represents hydrogen, methyl, ethyl, propyl, isopropyl, butyl, isobutyl, pentyl, hexyl, hydroxymethyl, hydroxyethyl, vinyl, allyl, ethynyl, fluorine, chlorine, bromine, iodine, bromovinyl, iodovinyl or trifluoromethyl; or B denotes triazoles of the general formula VI, wherein $R^2$ represents carboxamide $-CO-NH_2$, methoxycarbonyl $-COOCH_3$ or ethoxycarbonyl $-COOC_2H_5$; or B denotes imidazoles of the general formula VII, wherein one of the substituents $R^4$ and $R^5$ represents amino $-NH_2$ and the other represents carboxamide $-CO-N_2$, or cyano -CN.

9. Process according to any one of claims 1 - 8, in which isohexide nucleosides are prepared which are derivatives of the isomannide of the general formula Ia

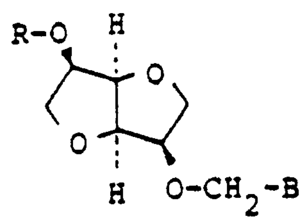

Ia

in which R and B have the meaning as defined in claims 1 - 8.

10. Process according to any one of claims 1 - 8, in which isohexide nucleosides are prepared which are derivatives of the isoidide of the general formula Ib

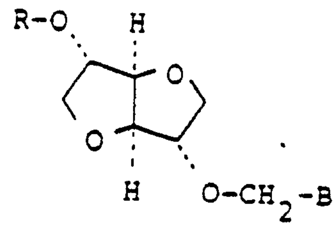

Ib

in which R and B have the meaning as defined in claims 1 - 8.

11. Process according to any one of claims 1 - 8, in which isohexide nucleosides are prepared which are derivatives of the isosorbide of the general formula Ic or Id

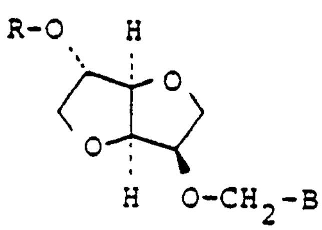

Ic

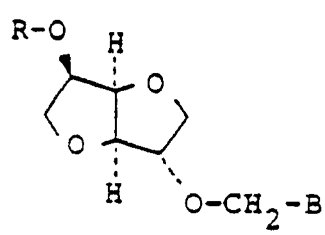

Id

in which R and B have the meaning as defined in claims 1 - 8.

12. Process according to any one of claims 1 - 4, in which 5-0-(3-Carbamoyl-1,2,4-triazol-l-yl-methyl)-

isosorbide is prepared.

**13.** Process according to any one of claims 1 - 4, in which 2-0-(3-Carbamoyl-1,2,4-triazol-l-yl-methyl)-isomannide is prepared.

**14.** Process according to any one of claims 1 - 4, in which 2-0-(5-Carbamoyl-1,2,4-triazol-l-yl-methyl)-isomannide is prepared.

**15.** The use of an isohexide nucleoside as prepared according to any one of claims 1 - 14 for manufacturing a medicament.

**16.** The use of an isohexide nucleoside as prepared according to any one of claims 1 - 14 for preventing and combating virus diseases.

## Revendications

**1.** Nucléosides d'isohexide de formule générale I

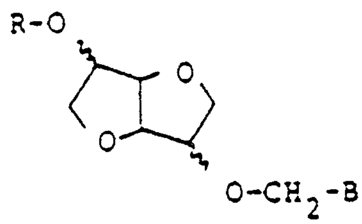

dans laquelle la liaison entre le système cyclique et les substituants peut être en position endocyclique ou exocyclique et dans laquelle

R

désigne l'hydrogène ; un groupe acyle aliphatique à chaîne droite ou à chaîne ramifiée ayant 2 à 5 atomes de carbone ; un groupe benzoyle facultativement substitué avec un halogène, un radical alkyle ayant jusqu'à 6 atomes de carbone ou un radical nitro ; un radical benzyle ou un radical phosphate ;

et B

représente un radical hétérocyclique contenant de l'azote choisi dans le groupe comprenant

a) des uraciles de formule générale II

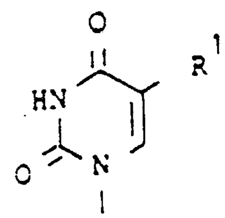

II

dans laquelle $R^1$ désigne l'hydrogène ou un halogène ou un groupe alkyle en $C_1$ à $C_6$ à chaîne droite ou ramifiée, vinyle, allyle ou éthynyle qui peut être substitué par des groupes hydroxyle ou par un ou plusieurs halogènes ;

b) des cytosines de formule générale III

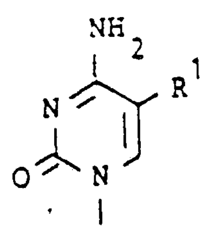

III

dans laquelle $R^1$ a la définition indiquée ci-dessus ;
c) des isocytosines de formule générale IV

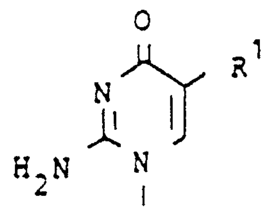

IV

dans laquelle $R^1$ a la définition indiquée ci-dessus ;
d) la 5-azacytosine de formule V

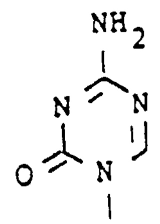

V

e) des triazoles de formule générale VI

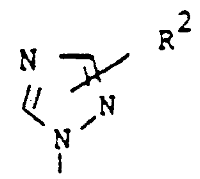

VI

dans laquelle $R^2$ occupe la position 3 ou la position 5 et peut avoir l'une des définitions suivantes : hydrogène ; groupe alkoxycarbonyle -$COOR^3$ dans lequel $R^3$ est un radical alkyle en $C_1$ à $C_5$ à chaîne droite ou ramifiée ; carboxamide -CO-$NH_2$ ; thiocarboxamide -CS-$NH_2$ ou cyano -CN ;
f) des imidazoles de formule générale VII

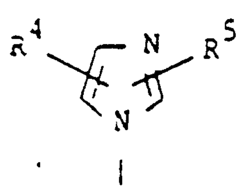

VII

dans laquelle les deux substituants $R^4$ et $R^5$ sont identiques ou différents et désignent l'hydrogène ou des substituants amino, -$NH_2$, carboxamide -CO-$NH_2$, thiocarboxamide -CS-$NH_2$, cyano -CN, ou, dans les cas appropriés, un sel de ces composés avec un acide inorganique ou organique.

**2.** Nucléosides d'isohexide suivant la revendication 1, dans lesquels R désigne un groupe acétyle.

**3.** Nucléosides d'isohexide suivant la revendication 1, dans lesquels R désigne un groupe benzoyle, toluoyle, chlorobenzoyle ou nitrobenzoyle.

**4.** Nucléosides d'isohexide suivant la revendication 1, dans lesquels R représente l'hydrogène ou un radical phosphate.

**5.** Nucléosides d'isohexide suivant la revendication 1, dans lequel R représente l'hydrogène ou un radical phosphate et B désigne des uraciles de formule générale II, des cytosines de formule générale III ou des isocytosines de formule générale IV, $R^1$ dans chaque cas représente l'hydrogène, un groupe

méthyle, éthyle, propyle, isopropyle, butyle, isobutyle, pentyle, hexyle, hydroxyméthyle, hydroxyéthyle, vinyle, allyle, éthynyle, le fluor, le chlore, le brome, l'iode, un groupe bromovinyle, iodovinyle ou trifluorométhyle ; ou bien B désigne des triazoles de formule générale VI, dans laquelle $R^2$ représente un carboxamide $-CO-NH_2$, méthoxycarbonyle $-COOCH_3$ ou éthoxycarbonyle $-COOC_2H_5$ ; ou bien B désigne des imidazoles de formule générale VII, dans laquelle l'un des substituants $R^4$ et $R^5$ est un groupe amino $-NH_2$ et les autres représentent un groupe carboxamide $-CO-NH_2$ ou un groupe cyano -CN.

6. Nucléosides d'isohexide suivant l'une des revendications 1 à 5, qui sont des dérivés de l'isomannide de formule générale Ia

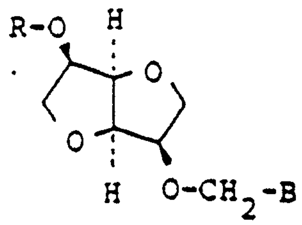

I a

dans laquelle R et B ont les définitions indiquées dans les revendications 1 à 5.

7. Nucléosides d'isohexide suivant l'une des revendications 1 à 5, qui sont des dérivés de l'isoidide de formule générale Ib

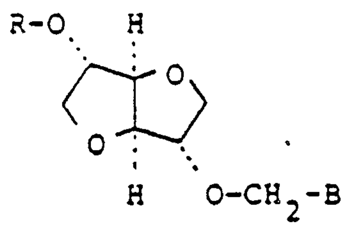

I b

dans laquelle R et B ont la définition indiquée dans les revendications 1 à 5.

8. Nucléosides d'isohexide suivant l'une des revendications 1 à 5, qui sont des dérivés de l'isosorbide de formule générale Ic ou Id

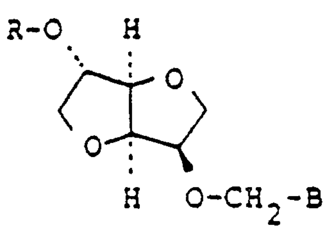

Ic

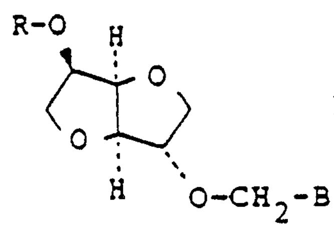

Id

dans laquelle R et B ont les définitions données dans les revendications 1 à 5.

9. Nucléoside d'isohexide suivant la revendication 1, qui est le 5-0-(3-carbamoyl-1,2,4-triazole-l-yl-méthyl) -isosorbide.

10. Nucléoside d'isohexide suivant la revendication 1, qui est le 2-0-(3-carbamoyl-1,2,4-triazole-l-yl-méthyl)-isomannide.

11. Nucléoside d'isohexide suivant la revendication 1, qui est le 2-0-(5-carbamoyl-1,2,4-triazole-l-yl-méthyl)-isommanide.

**12.** Procédé de préparation d'un nucléoside d'isohexide suivant la revendication 1, qui consiste, d'une manière connue et conformément à l'équation suivante

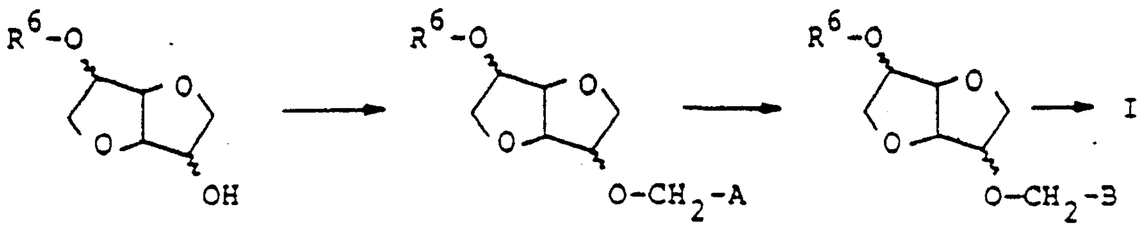

VIII IX X

à convertir un isohexide monosubstitué de formule générale VIII dans laquelle $R^6$ a la même définition que R, facultativement protégé par un groupe protecteur d'emploi courant dans la chimie des nucléosides, en un composé de formule générale IX dans laquelle A représente un groupe partant ou un atome partant, et à convertir le composé de formule générale IX en un composé de formule générale X soit par réaction de ce composé avec une base hétérocyclique contenant de l'azote, de formule générale XI

$$B - H \qquad XI$$

dans laquelle B a la définition mentionnée ci-dessus, soit par sa réaction avec un dérivé protégé de cette base hétérocyclique contenant de l'azote, et, si nécessaire, à détacher le groupe protecteur de $R^6$ dans le composé de formule générale X et, le cas échéant, à convertir le composé de formule générale I, dans laquelle R désigne l'hydrogène, en un composé de formule générale I dans laquelle R désigne un radical phosphate par réaction avec un réactif de phosphorylation, de préférence avec l'oxytrichlorure de phosphore.

**13.** Utilisation d'un nucléoside d'isohexide suivant l'une quelconque des revendications 1 à 11 pour la préparation d'un médicament.

**14.** Utilisation d'un nucléoside d'isohexide suivant l'une quelconque des revendications 1 à 11 pour la préparation d'un médicament destiné à prévenir ou à combattre des maladies virales.

**15.** Médicament contenant au moins un composé suivant l'une quelconque des revendications 1 à 11 et des adjuvants et/ou des excipients usuels.

Revendications pour les Etats contractants suivants : ES, GR

**1.** Procédé de préparation de nucléosides d'isohexide de formule générale I

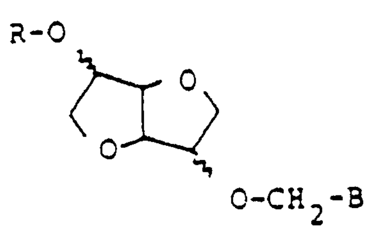

I

dans laquelle la liaison entre le système cyclique et les substituants peut être en position endocyclique ou exocyclique et dans laquelle

R

désigne l'hydrogène ; un groupe acyle aliphatique à chaîne droite ou à chaîne ramifiée ayant 2 à 5 atomes de carbone ; un groupe benzoyle facultativement substitué avec un halogène, un radical alkyle ayant jusqu'à 6 atomes de carbone ou un radical nitro ; un radical benzyle ou un radical phosphate ;

et B

représente un radical hétérocyclique contenant de l'azote choisi dans le groupe comprenant

a) des uraciles de formule générale II

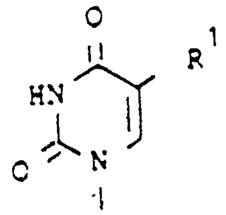

. II

dans laquelle $R^1$ désigne l'hydrogène ou un halogène ou un groupe alkyle en $C_1$ à $C_6$ à chaîne droite ou ramifiée, vinyle, allyle ou éthynyle qui peut être substitué par des groupes hydroxyle ou par un ou plusieurs halogènes ;

b) des cytosines de formule générale III

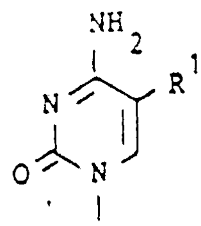

III

dans laquelle $R^1$ a la définition indiquée ci-dessus ;

c) des isocytosines de formule générale IV

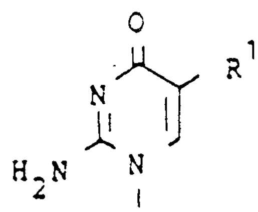

IV

dans laquelle $R^1$ a la définition indiquée ci-dessus ;

d) la 5-azacytosine de formule V

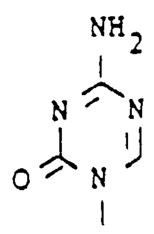

V

e) des triazoles de formule générale VI

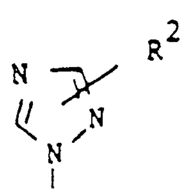

VI

dans laquelle $R^2$ occupe la position 3 ou la position 5 et peut avoir l'une des définitions suivantes : hydrogène ; qroupe alkoxycarbonyle $-COOR^3$ dans lequel $R^3$ est un radical alkyle en $C_1$ à $C_5$ à chaîne droite ou ramifiée ; carboxamide $-CO-NH_2$ ; thiocarboxamide $-CS-NH_2$ ou cyano $-CN$ ;

f) des imidazoles de formule générale VII

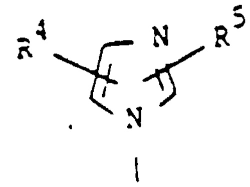

VII

dans laquelle les deux substituants $R^4$ et $R^5$ sont identiques ou différents et désignent l'hydrogène ou des substituants amino, $-HH_2$, carboxamide $-CO-NH_2$, thiocarboxamide $-CS-NH_2$, cyano -CN, ou, dans les cas appropriés, un sel de ces composés avec un acide inorganique ou organique, procédé qui consiste d'une manière connue et conformément à l'équation suivante

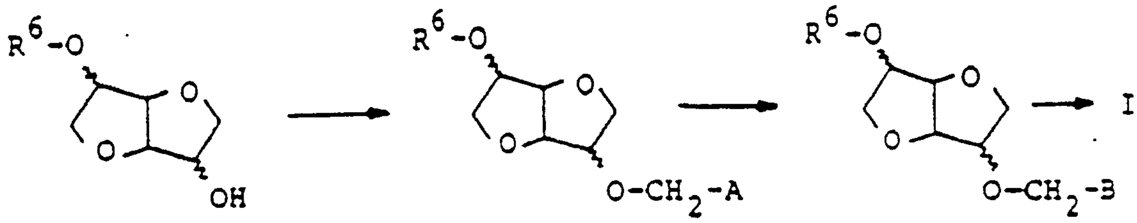

VIII IX X

à convertir un isohexide monosubstitué de formule générale VIII dans laquelle $R^6$ a la même définition que R, facultativement protégé par un groupe protecteur d'emploi courant dans la chimie des nucléosides, en un composé de formule générale IX dans laquelle A représente un groupe partant ou un atome partant, et à convertir le composé de formule générale IX en un composé de formule générale X soit par réaction de ce composé avec une base hétérocyclique contenant de l'azote, de formule générale XI

$$B - H \qquad \text{XI}$$

dans laquelle B a la définition mentionnée ci-dessus, soit par sa réaction avec un dérivé protégé de cette base hétérocyclique contenant de l'azote, et, si nécessaire, à détacher le groupe protecteur de $R^6$ dans le composé de formule générale X et, le cas échéant, à convertir le composé de formule générale I, dans laquelle R désigne l'hydrogène, en un composé de formule générale I dans laquelle R désigne un radical phosphate par réaction avec un réactif de phosphorylation, de préférence avec l'oxytrichlorure de phosphore.

**2.** Procédé suivant la revendication 1, dans lequel A représente le chlore, le brome ou l'iode ou un radical acyloxy aliphatique en $C_2$ à $C_5$ à chaîne droite ou à chaîne ramifiée.

**3.** Procédé suivant la revendication 1 ou 2, dans lequel le groupe protecteur de la base hétérocyclique contenant de l'azote, de formule générale XI, comprend des radicaux acyle, aliphatiques ou aromatiques, de préférence acétyle ou benzoyle.

**4.** Procédé suivant la revendication 1 ou 2, dans lequel le composé de formule IX est amené à réagir avec un dérivé silylé de la base hétérocyclique contenant de l'azote, un dérivé silylé étant un dérivé que l'on obtient par réaction de la base de formule générale XI avec un halogénure de trialkylsilyle, de préférence le chlorure de triméthylsilyle, ou un hexa-alkyldisilazane, de préférence l'hexaméthyldisilazane, ou avec un mélange des deux, ou est amené à réagir avec un sel métallique de la base hétérocyclique contenant de l'azote, de préférence un sel d'argent ou de mercure, ou le cas échéant, est amené à réagir avec un dérivé alkoxylé de cette base hétérocyclique contenant de l'azote, le terme "alkoxy" étant utilisé pour désigner un groupe alkoxy inférieur, de préférence un groupe méthoxy ou éthoxy.

5. Procédé suivant l'une quelconque des revendications 1 à 4, dans lequel on prépare un nucléoside d'isohexide de formule générale I dans laquelle R désigne un groupe acétyle.

6. Procédé suivant l'une quelconque des revendications 1 à 4, dans lequel on prépare un nucléoside d'isohexide de formule générale I dans laquelle R désigne un groupe benzoyle, toluoyle, chlorobenzoyle, nitrobenzoyle.

7. Procédé suivant l'une quelconque des revendications 1 à 4, dans lequel on prépare un nucléoside d'isohexide de formule générale I dans laquelle R représente l'hydrogène ou un radical phosphate.

8. Procédé suivant l'une quelconque des revendications 1 à 4, dans lequel on prépare un nucléoside d'isohexide de formule générale I dans laquelle R représente l'hydrogène ou un radical phosphate et B désigne des uraciles de formule générale II, des cytosines de formule générale III ou des isocytosines de formule générale IV, $R^1$ dans chaque cas représente l'hydrogène, un groupe méthyle, éthyle, propyle, isopropyle, butyle, isobutyle, pentyle, hexyle, hydroxyméthyle, hydroxyéthyle, vinyle, allyle, éthynyle, le fluor, le chlore, le brome, l'iode, un groupe bromovinyle, iodovinyle ou trifluorométhyle ; ou bien B désigne des triazoles de formule générale VI, dans laquelle $R^2$ représente un carboxamide $-CO-NH_2$, méthoxycarbonyle $-COOCH_3$ ou éthoxycarbonyle $-COOC_2H_5$ ; ou bien B désigne des imidazoles de formule générale VII, dans laquelle l'un des substituants $R^4$ et $R^5$ est un groupe amino $-NH_2$ et les autres représentent un groupe carboxamide $-CO-NH_2$ ou un groupe cyano -CN.

9. Procédé suivant l'une quelconque des revendications 1 à 8, dans lequel on prépare des nucléosides d'isohexide qui sont des dérivés de l'isomannide de formule générale Ia

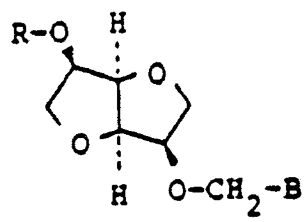

I a

dans laquelle R et B ont les définitions données dans les revendications 1 à 8.

10. Procédé suivant l'une quelconque des revendications 1 à 8, dans lequel on prépare des nucléosides d'isohexide qui sont des dérivés de l'isoidide de formule générale Ib

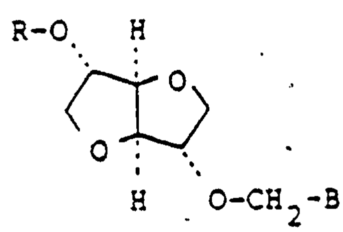

I b

dans laquelle R et B ont les définitions indiquées dans les revendications 1 à 8.

11. Procédé suivant l'une quelconque des revendications 1 à 8, dans lequel on prépare des nucléosides d'isohexide qui sont des dérivés de l'isosorbide de formule générale Ic ou Id

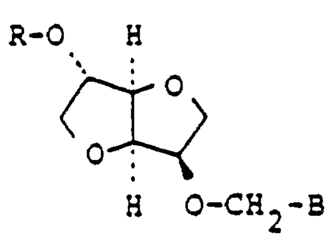

Ic

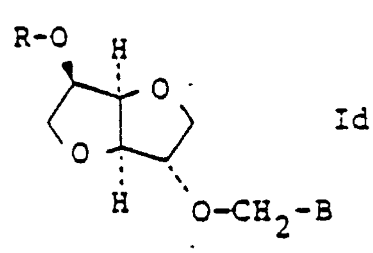

Id

dans laquelle R et B ont les définitions données dans les revendications 1 à 8.

**12.** Procédé suivant l'une quelconque des revendications 1 à 4, dans lequel on prépare le 5-0-(3-carbamoyl-1,2,4-triazole-l-yl-méthyl)-isosorbide.

**13.** Procédé suivant l'une quelconque des revendications 1 à 4, dans lequel on prépare le 2-0-(3-carbamoyl-1,2,4-triazole-l-yl-méthyl)-isomannide.

**14.** procédé suivant l'une quelconque des revendications 1 à 4, dans lequel on prépare le 2-0-(5-carbamoyl-1,2,4-triazole-l-yl-méthyl)-isomannide.

**15.** Utilisation d'un nucléoside d'isohexide tel qu'obtenu suivant l'une quelconque des revendications 1 à 14 pour la préparation d'un médicament.

**16.** Utilisation d'un nucléoside d'isohexide tel qu'obtenu conformément à l'une quelconque des revendications 1 à 14 pour prévenir et combattre des maladies virales.

**Ansprüche**

**1.** Isohexidnukleoside der allgemeinen Formel (I)

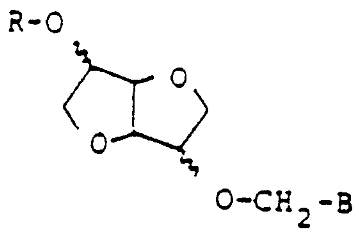

(I),

worin die Bindung zwischen dem Ringsystem und den Substituenten in der Stellung entweder endo- oder exocyclisch sein kann und worin

R Wasserstoff; geradkettiges oder verzweigtes aliphatisches Acyl mit 2 bis 5 Kohlenstoffatomen; Benzoyl gegebenenfalls substituiert mit Halogen, Alkyl mit bis zu 6 Kohlenstoffatomen oder Nitro; Benzyl oder einen Phosphatrest bedeutet; und

B einen stickstoffhaltigen heterocyclischen Rest darstellt ausgewählt aus der Gruppe bestehend aus

a) Uracilen der allgemeinen Formel (II)

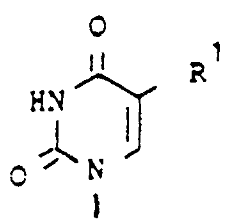

(II),

worin $R^1$ Wasserstoff, Halogen, geradkettiges oder verzweigtes $C_1$-$C_8$-Alkyl, Vinyl, Allyl oder Äthinyl bedeutet, das durch Hydroxylgruppen oder ein oder mehrere Halogene substituiert sein kann;

b) Cytosinen der allgemeinen Formel (III)

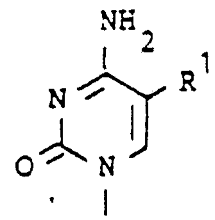

(III),

worin $R^1$ die obige Bedeutung hat;

c) Isocytosinen der allgemeinen Formel (IV)

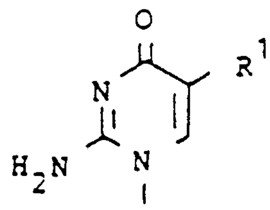

(IV),

worin $R^1$ die obige Bedeutung hat;

d) 5-Azacytosin der Formel (V)

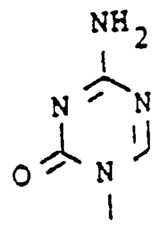

(V)

e) Triazolen der allgemeinen Formel (VI)

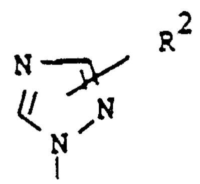

(VI),

worin $R^2$ entweder die 3- oder die 5-Stellung einnimmt und eine der folgenden Bedeutungen haben kann: Wasserstoff; Alkoxycarbonyl $-COOR^3$, wobei $R^3$ geradkettiges oder verzweigtes $C_1$-$C_5$-Alkyl darstellt; Carboxamid $-CO-NH_2$; Thiocarboxamid $-CS-NH_2$ oder Cyano -CN;

f) Imidazolen der allgemeinen Formel (VII)

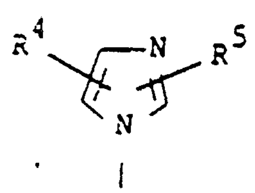

(VII),

worin die beiden Substituenten $R^4$ und $R^5$ gleich oder verschieden sind und Wasserstoff, Amino $-NH_2$, Carboxamid $-CO-NH_2$, Thiocarboxamid $-CS-NH_2$ oder Cyano -CN darstellen oder, wenn geeignet, ein Salz hievon mit einer anorganischen oder organischen Säure.

**2.** Isohexidnukleoside nach Anspruch 1, worin R Acetyl bedeutet.

**3.** Isohexidnukleoside nach Anspruch 1, worin R Benzoyl, Toluoyl, Chlorbenzoyl oder Nitrobenzoyl bedeutet.

**4.** Isohexidnukleoside nach Anspruch 1, worin R Wasserstoff oder einen Phosphatrest bedeutet.

**5.** Isohexidnukleoside nach Anspruch 1, worin R Wasserstoff oder einen Phosphatrest bedeutet und B Uracile der allgemeinen Formel (II), Cytosine der allgemeinen Formel (III) oder Isocytosine der allgemeinen Formel (IV) darstellt, wobei $R^1$ in jedem Fall Wasserstoff, Methyl, Äthyl, Propyl, Isopropyl, Butyl, Isobutyl, Pentyl, Hexyl, Hydroxymethyl, Hydroxyäthyl, Vinyl, Allyl, Äthinyl, Fluor, Chlor, Brom, Jod, Bromvinyl, Jodvinyl oder Trifluormethyl ist; oder B Triazole der allgemeinen Formel (VI) darstellt, wobei $R^2$ Carboxamid $-CO-NH_2$, Methoxycarbonyl $-COOCH_3$ oder Äthoxycarbonyl $-COOC_2H_5$ ist; oder B Imidazole der allgemeinen Formel (VII) darstellt, wobei einer der Substituenten $R^4$ und $R^5$ Amino $-NH_2$ ist und der andere Carboxamid $-CO-N_2$ oder Cyano $-CN$ darstellt.

**6.** Isohexidnukleoside nach einem der Ansprüche 1 bis 5, die Derivate des Isomannids der allgemeinen Formel (Ia)

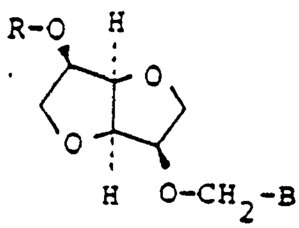

(Ia)

sind, worin R und B die in den Ansprüchen 1 bis 5 angegebene Bedeutung haben.

**7.** Isohexidnukleoside nach einem der Ansprüche 1 bis 5, die Derivate des Isoidids der allgemeinen Formel (Ib)

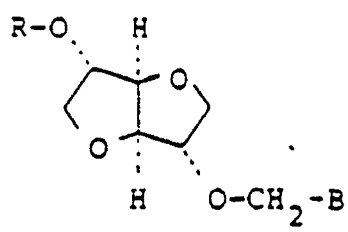

(Ib)

sind, worin R und B die in den Ansprüchen 1 bis 5 angegebene Bedeutung haben.

**8.** Isohexidnukleoside nach einem der Ansprüche 1 bis 5, die Derivate des Isosorbids der allgemeinen Formel (Ic) oder (Id)

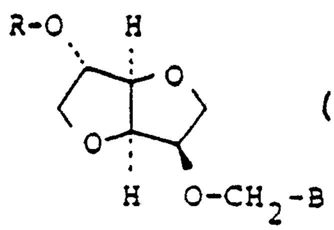

(Ic)

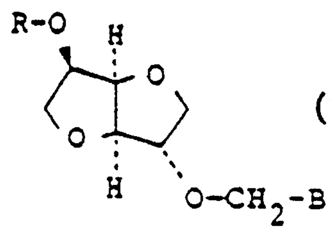

(Id)

sind, worin R und B die in den Ansprüchen 1 bis 5 angegebene Bedeutung haben.

**9.** Isohexidnukleosid nach Anspruch 1, das 5-0-(3-Carbamoyl-1,2,4-triazol-1-yl-methyl)-isosorbid ist.

**10.** Isohexidnukleosid nach Anspruch 1, das 2-0-(3-Carbamoyl-1,2,4-triazol-1-yl-methyl)-isomannid ist.

**11.** Isohexidnukleosid nach Anspruch 1, das 2-0-(5-Carbamoyl-1,2,4-triazol-1-yl-methyl)-isomannid ist.

**12.** Verfahren zur Herstellung eines Isohexidnuclosids nach Anspruch 1, das, in an sich bekannter Weise, gemäß der folgenden Gleichung

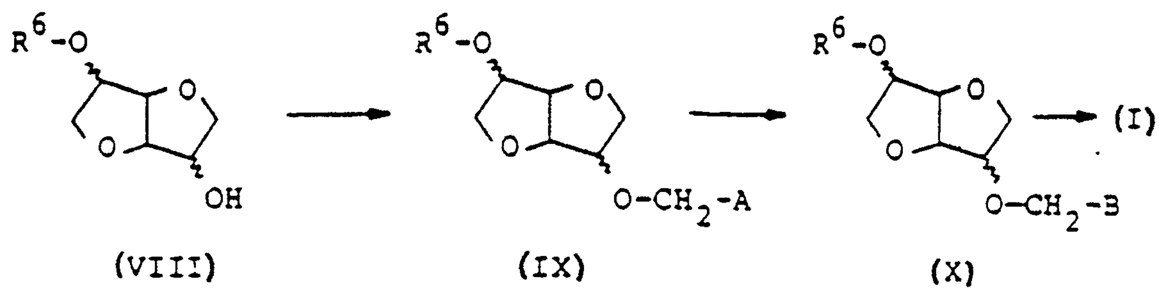

das Überführen eines monosubstituierten Isohexids der allgemeinen Formel (VIII), worin $R^6$ die gleiche Bedeutung wie R hat, gegebenenfalls geschützt durch eine in der Nukleosidchemie übliche Schutzgruppe, in eine Verbindung der allgemeinen Formel (IX), worin A eine abspaltbare Gruppe oder ein abspaltbares Atom ist, und das Überführen der Verbindung der allgemeinen Formel (IX) in eine Verbindung der allgemeinen Formel (X) entweder durch Umsetzen mit einer stickstoffhaltigen heterocyclischen Base der allgemeinen Formel (XI)

B - H (XI),

worin B die obige Bedeutung hat, oder durch Umsetzen mit einem geschützten Derivat dieser stickstoffhaltigen Base und, wenn geeignet, das Abspalten der Schutzgruppe von $R^6$ in der Verbindung der allgemeinen Formel (X) und, wenn geeignet, das Überführen der Verbindung der allgemeinen Formel (I), worin R Wasserstoff bedeutet, in eine Verbindung der allgemeinen Formel (I), worin R einen Phosphatrest darstellt, durch Umsetzen mit einem Phosphorylierungsmittel, vorzugsweise mit Phosphoroxytrichlorid, umfaßt.

**13.** Verwendung eines Isohexidnukleosids nach einem der Ansprüche 1 bis 11 zur Herstellung eines Medikaments.

**14.** Verwendung eines Isohexidnukleosids nach einem der Ansprüche 1 bis 11 zur Herstellung eines Medikaments zur Verhütung und Bekämpfung von Viruserkrankungen.

**15.** Medikament, das mindestens eine Verbindung nach einem der Ansprüche 1 bis 11 und übliche Hilfsstoffe und/oder Exzipienten enthält.

Patentansprüche für folgende Vertragsstaaten : ES, GR

**1.** Verfahren zur Herstellung von Isohexidnukleosiden der allgemeinen Formel (I)

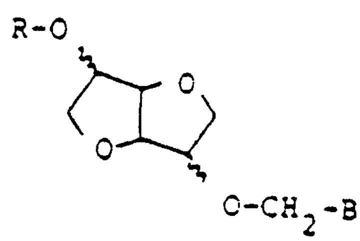

(I),

worin die Bindung zwischen dem Ringsystem und den Substituenten in der Stellung entweder endo- oder exocyclisch sein kann und worin

R Wasserstoff; geradkettiges oder verzweigtes aliphatisches Acyl mit 2 bis 5 Kohlenstoffatomen;

Benzoyl gegebenenfalls substituiert mit Halogen, Alkyl mit bis zu 6 Kohlenstoffatomen oder Nitro; Benzyl oder einen Phosphatrest bedeutet; und

B einen stickstoffhaltigen heterocyclischen Rest darstellt ausgewählt aus der Gruppe bestehend aus

a) Uracilen der allgemeinen Formel (II)

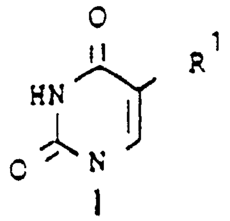

(II),

worin $R^1$ Wasserstoff, Halogen, geradkettiges oder verzweigtes $C_1$-$C_6$-Alkyl, Vinyl, Allyl oder Äthinyl bezeichnet, das durch Hydroxylgruppen oder ein oder mehrere Halogene substituiert sein kann;

b) Cytosinen der allgemeinen Formel (III)

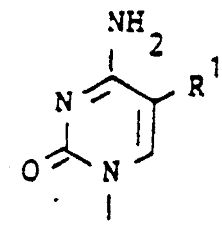

(III),

worin $R^1$ die obige Bedeutung hat;

c) Isocytosinen der allgemeinen Formel (IV)

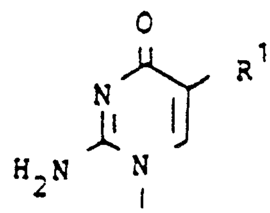

(IV),

worin $R^1$ die obige Bedeutung hat;

d) 5-Azacytosin der Formel (V)

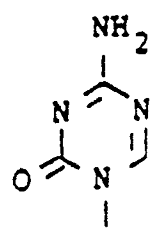

(V)

e) Triazolen der allgemeinen Formel (VI)

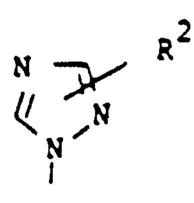

(VI),

worin $R^2$ entweder die 3- oder die 5-Stellung einnimmt und eine der folgenden Bedeutungen haben kann: Wasserstoff; Alkoxycarbonyl $-COOR^3$, wobei $R^3$ geradkettiges oder verzweigtes $C_1$-$C_5$-Alkyl darstellt; Carboxamid $-CO-NH_2$; Thiocarboxamid $-CS-NH_2$ oder Cyano -CN;
f) Imidazolen der allgemeinen Formel (VII)

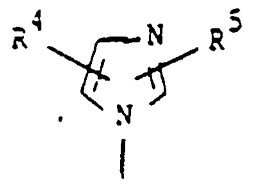

(VII),

worin die beiden Substituenten $R^4$ und $R^5$ gleich oder verschieden sind und Wasserstoff, Amino $-NH_2$, Carboxamid $-CO-NH_2$, Thiocarboxamid $-CS-NH_2$ oder Cyano -CN darstellen oder, wenn geeignet, eines Salz hievon mit einer anorganischen oder organischen Säure, welches Verfahren, in an sich bekannter Weise, gemäß der folgenden Gleichung

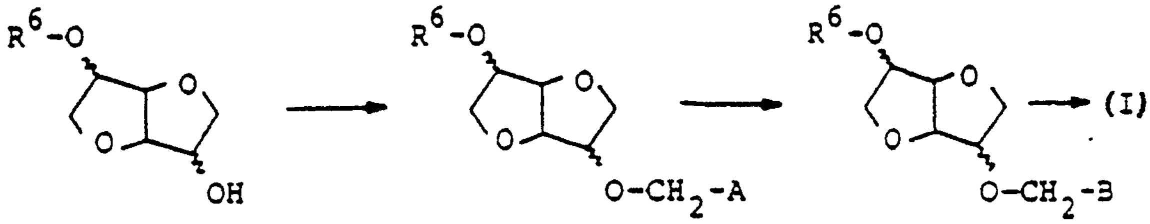

(VIII) (IX) (X)

das Überführen eines monosubstituierten Isohexids der allgemeinen Formel (VIII), worin $R^6$ die gleiche Bedeutung wie R hat, gegebenenfalls geschützt durch eine in der Nukleosidchemie übliche Schutzgruppe, in eine Verbindung der allgemeinen Formel (IX), worin A eine abspaltbare Gruppe oder ein abspaltbares Atom ist, und das Überführen der Verbindung der allgemeinen Formel (IX) in eine Verbindung der allgemeinen Formel (X) entweder durch Umsetzen mit einer stickstoffhaltigen heterocyclischen Base der allgemeinen Formel (XI)

$$B - H \qquad (XI),$$

worin B die obige Bedeutung hat, oder durch Umsetzen mit einem geschützten Derivat dieser stickstoffhaltigen Base und, wenn geeignet, das Abspalten der Schutzgruppe von $R^6$ in der Verbindung der allgemeinen Formel (X) und, wenn geeignet, das Überführen der Verbindung der allgemeinen Formel (I), worin R Wasserstoff bedeutet, in eine Verbindung der allgemeinen Formel (I), worin R einen Phosphatrest darstellt, durch Umsetzen mit einem Phosphorylierungsmittel, vorzugsweise mit Phosphoroxytrichlorid, umfaßt.

2. Verfahren nach Anspruch 1, worin A Chlor, Brom oder Jod oder einen geradkettigen oder verzweigten aliphatischen $C_2$-$C_5$-Acyloxyrest bedeutet.

3. Verfahren nach Anspruch 1 oder 2, worin die Schutzgruppe in der stickstoffhaltigen heterocyclischen Base der allgemeinen Formel (XI) aliphatische oder aromatische Acylreste, vorzugsweise Acetyl oder Benzoyl, umfaßt.

4. Verfahren nach Anspruch 1 oder 2, worin die Verbindung der Formel (IX) mit einem silylierten Derivat der stickstoffhaltigen heterocyclischen Base umgesetzt wird, wobei unter einem silylierten Derivat ein solches verstanden wird, das durch Umsetzen der Base der allgemeinen Formel (XI) mit einem Trialkylsilylhalogenid, vorzugsweise Trimethylsilylchlorid, oder einem Hexaalkyldisilazan, vorzugsweise Hexamethyldisilazan, oder mit einer Mischung der beiden erhalten wird, oder mit einem Metallsalz der stickstoffhaltigen heterocyclischen Base, vorzugsweise einem Silber- oder Quecksilbersalz, umgesetzt

wird oder, wenn geeignet, mit einem Alkoxyderivat dieser stickstoffhaltigen heterocyclischen Base umgesetzt wird, wobei unter "Alkoxy" eine nied.Alkoxygruppe, vorzugsweise eine Methoxy- oder Äthoxygruppe verstanden wird.

5. Verfahren nach einem der Ansprüche 1 bis 4, worin ein Isohexidnukleosid der allgemeinen Formel (I) hergestellt wird, worin R Acetyl bedeutet.

6. Verfahren nach einem der Ansprüche 1 bis 4, worin ein Isohexidnukleosid der allgemeinen Formel (I) hergestellt wird, worin R Benzoyl, Toluoyl, Chlorbenzoyl oder Nitrobenzoyl bedeutet.

7. Verfahren nach einem der Ansprüche 1 bis 4, worin ein Isohexidnukleosid der allgemeinen Formel (I) hergestellt wird, worin R Wasserstoff oder einen Phosphatrest bedeutet.

8. Verfahren nach einem der Ansprüche 1 bis 4, worin ein Isohexidnukleosid der allgemeinen Formel (I) hergestellt wird, worin R Wasserstoff oder einen Phosphatrest bedeutet und B Uracile der allgemeinen Formel (II), Cytosine der allgemeinen Formel (III) oder Isocytosine der allgemeinen Formel (IV) darstellt, wobei $R^1$ in jedem Fall Wasserstoff, Methyl, Äthyl, Propyl, Isopropyl, Butyl, Isobutyl, Pentyl, Hexyl, Hydroxymethyl, Hydroxyäthyl, Vinyl, Allyl, Äthinyl, Fluor, Chlor, Brom, Jod, Bromvinyl, Jodvinyl oder Trifluormethyl ist; oder B Triazole der allgemeinen Formel (VI) darstellt, worin $R^2$ Carboxamid $-CO-NH_2$, Methoxycarbonyl $-COOCH_3$ oder Äthoxycarbonyl $-COOC_2H_5$ ist; oder B Imidazole der allgemeinen Formel (VII) darstellt, wobei einer der Substituenten $R^4$ und $R^5$ Amino $-NH_2$ ist und der andere Carboxamid $-CO-N_2$ oder Cyano -CM darstellt.

9. Verfahren nach einem der Ansprüche 1 bis 8, worin Isohexidnukleoside hergestellt werden, die Derivate des Isomannids der allgemeinen Formel (Ia)

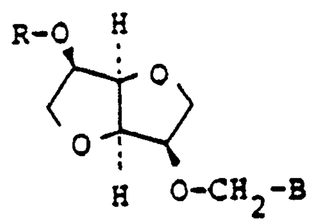

(Ia)

sind, worin R und B die in den Ansprüchen 1 bis 8 angegebene Bedeutung haben.

10. Verfahren nach einem der Ansprüche 1 bis 8, worin Isohexidnukleoside hergestellt werden, die Derivate des Isoidids der allgemeinen Formel (Ib)

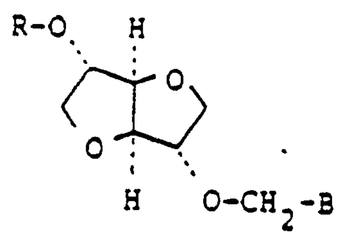

(Ib)

sind, worin R und B die in den Ansprüchen 1 bis 8 angegebene Bedeutung haben.

11. Verfahren nach einem der Ansprüche 1 bis 8, worin Isohexidnukleoside hergestellt werden, die Derivate des Isosorbids der allgemeinen Formel (Ic) oder (Id)

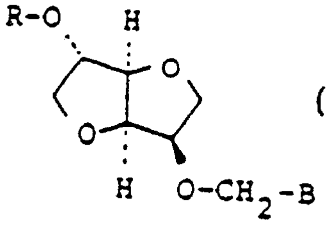

(Ic)

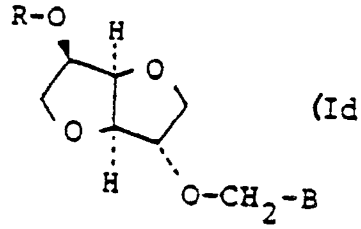

(Id)

sind, worin R und B die in den Ansprüchen 1 bis 8 angegebene Bedeutung haben.

**12.** Verfahren nach einem der Ansprüche 1 bis 4, worin 5-0-(3-Carbamoyl-1,2,4-triazol-l-yl-methyl)-isosorbid hergestellt wird.

**13.** Verfahren nach einem der Ansprüche 1 bis 4, worin 2-0-(3-Carbamoyl-1,2,4-triazol-l-yl-methyl)-isomannid hergestellt wird.

**14.** Verfahren nach einem der Ansprüche 1 bis 4, worin 2-0-(5-Carbamoyl-1,2,4-triazol-l-yl-methyl)-isomannid hergestellt wird.

**15.** Verwendung eines Isohexidnukleosids, hergestellt nach einem der Ansprüche 1 bis 14, zur Herstellung eines Medikaments.

**16.** Verwendung eines Isohexidnukleosids, hergestellt nach einem der Ansprüche 1 bis 14, zur Verhütung und Bekämpfung von Viruserkrankungen.